# FASCICULE DE BREVET EUROPEEN

(11) **EP 2 939 020 B1**
(45) Date de publication et mention de la délivrance du brevet: **08.05.2019**
(21) Numéro de dépôt: 13827024.4
(22) Date de dépôt: 27.12.2013
(51) Int. Cl.: G01N 33/48, B01L 3/00

(54) **DISPOSITIF ADAPTÉ POUR RECEVOIR UN ÉCHANTILLON BIOLOGIQUE**
VORRICHTUNG ZUR AUFNAHME EINER BIOLOGISCHEN PROBE
DEVICE DESIGNED TO RECEIVE A BIOLOGICAL SAMPLE

(30) Priorité: 28.12.2012 FR 1262966
(43) Date de publication de la demande: 04.11.2015
(73) Titulaire: bioMérieux, 69280 Marcy-l'Etoile (FR)
(72) Inventeur: COLIN, Bruno, F-69280 Marcy l'Etoile (FR); MONTET, Marie-Pierre, F-69290 Grézieu la Varenne (FR); ROZAND, Christine, F-69280 St Genis les Ollières (FR)
(74) Mandataire: Murgitroyd & Company
(86) Numéro de dépôt international: PCT/FR2013/053277
(87) Numéro de publication internationale: WO 2014/102517

(56) Documents cités:
- EP-A2- 0 378 353
- WO-A1-2012/004540
- FR-A1- 2 612 297
- US-A- 5 335 673

## Description

### Domaine technique

La présente invention concerne, de manière générale, l'analyse et/ou le transfert aux fins d'analyse d'échantillons biologiques. De manière générale, la présente invention vise à réduire les risques de contamination de l'environnement et/ou du personnel dans le cadre d'analyses - et plus généralement de manipulations - d'échantillons biologiques, de préférence susceptibles/suspectés de contenir de la matière microbiologique.

### Etat de la technique

Dans le domaine de l'analyse, en particulier celui de l'analyse biologique (et plus particulièrement de l'analyse microbiologique), se pose régulièrement le problème de la sécurité du personnel technique qui manipule des échantillons biologiques, notamment à cause des risques de contamination existant lors du prélèvement d'un aliquote, en vue d'une analyse. Ceci est particulièrement vrai lorsque l'on utilise des dispositifs d'analyse, permettant par exemple de détecter un ou plusieurs micro-organisme(s) cible(s), hors laboratoire dans le cadre de technologies « point-of-care » (en langue anglaise).

Ainsi, l'on connaît les risques encourus par le personnel technique qui doit prélever un aliquote dans une poche de sang destinée à la transfusion, afin de vérifier l'innocuité de ce prélèvement. Les poches de sang sont loin de constituer un contenant facile à manipuler de par leurs parois souples. Il s'ensuit que les personnes qui manipulent ces poches de sang, peuvent très facilement entrer en contact avec le prélèvement et risquer de se contaminer si ce dernier n'est pas exempt d'agents pathogènes.

La contamination peut également se produire en sens inverse. En effet, il arrive que lors de prélèvements de liquides biologiques, tels que les urines, suspectés d'être contaminés, le personnel qui effectue le prélèvement contamine lui-même ce dernier, entraînant une impossibilité de fournir un diagnostic fiable à partir dudit prélèvement.

Dans le cas de prélèvements alimentaires à des fins de contrôle qualité, il est aussi très important de s'assurer qu'aucune contamination parasite n'est effectuée par le personnel technique qui est en charge de l'analyse.

En outre, il est fréquent de procéder à une mise en culture du prélèvement alimentaire qui a été réalisé. Cette mise en culture consiste généralement à introduire le prélèvement dans une poche en plastique contenant un milieu de culture qui permet le développement des micro-organismes, en particulier des bactéries. Après un temps d'incubation nécessaire au développement microbiologique, un ou plusieurs prélèvements aliquotes du milieu de culture sont réalisés, afin de mettre en oeuvre une analyse microbiologique. Les prélèvements aliquotes sont généralement réalisés à l'issue de la phase de pré-enrichissement/enrichissement en ouvrant la poche, au niveau de l'orifice d'introduction du prélèvement alimentaire et du milieu de culture. Un tel processus est souvent critique à mettre en oeuvre dans la mesure où le technicien chargé de cette démarche doit simultanément maintenir la poche ouverte, tenir le dispositif de fermeture de la poche, ainsi que la pipette qui lui sert à aspirer le milieu de culture. Par ailleurs, de telles conditions de manipulation augmentent sensiblement le temps d'analyse. Or, lorsque le nombre d'échantillons est important, la productivité peut en être sérieusement entamée.

Eu égard à ces problématiques, la Demanderesse a mis au point un procédé innovant et facilement automatisable pour la détection et l'identification de micro-organismes (ainsi que pour la confirmation de cette détection et/ou identification) qui permet notamment de limiter les manipulations de l'échantillon biologique contenu dans un contenant, limitant de ce fait les risques de contamination, soit du personnel manipulant l'échantillon, soit de l'échantillon *per se.* Ce procédé innovant est divulgué dans la demande internationale WO 2012/004540. Selon un mode de réalisation préféré, la détection du ou des micro-organisme(s) cible(s) s'effectue par lecture optique d'un support de capture sensibilisé avec au moins un partenaire de liaison spécifique du ou des micro-organisme(s) cible(s) à des fins de détection optique. Cette lecture optique du résultat, qui s'effectue à travers la paroi transparente du contenant (par exemple un sac ou une poche plastique), peut être réalisée visuellement ou par l'intermédiaire d'un dispositif de lecture optique.

Le procédé selon WO 2012/004540 permet un accès direct aux résultats d'analyse sans nécessiter de manipulation de la part du personnel technique. Il en résulte que ce procédé, ainsi que les dispositifs permettant la mise en oeuvre de ce dernier, peuvent être utilisés hors laboratoire, sur le terrain, afin de détecter, par exemple, une contamination microbienne d'un échantillon biologique notamment dans le cadre d'analyses médicales ou agroalimentaires. En conséquence, il importe de limiter les risques de contamination microbienne pour l'utilisateur et/ou son environnement, liés par exemple à une ouverture accidentelle du contenant (pouvant résulter d'une rupture ou déchirure d'une paroi dudit contenant) ou encore d'une ouverture inopportune conséquente à une erreur de manipulation.

En outre, lors d'une utilisation hors laboratoire (technologie « point-of-care » en langue anglaise) il est important de pouvoir assurer une stérilisation optimale du contenant et de son contenu (incluant le support de capture susceptible de contenir les micro-organismes cibles), et ce en l'absence de dispositifs de stérilisation de type autoclaves (communément employés dans les laboratoires).

Par ailleurs, à l'issue du procédé divulgué dans la demande internationale WO 2012/004540, la Demanderesse a découvert qu'il pouvait s'avérer extrêmement intéressant de récupérer le susdit support de capture susceptible de contenir les micro-organismes cibles (et éventuellement une partie de l'échantillon biologique) en vue d'analyses ultérieures, par exemple aux fins de confirmation. Le transfert dudit support de capture (et éventuellement d'une partie de l'échantillon biologique) doit pouvoir être effectué sans risque de contamination pour l'utilisateur et/ou l'environnement.

EP 0 378 353 A2 divulgue un dispositif permettant de recueillir des fluides biologiques et de contenir un échantillon prédéterminé pour l'analyser.

L'invention vise donc à résoudre tout ou partie des problèmes susvisés.

### Exposé de l'invention

En conséquence, un objet de l'invention concerne un dispositif adapté pour recevoir au moins un échantillon biologique, ledit dispositif comprenant :
- au moins un contenant, de préférence étanche, adapté pour recevoir ledit échantillon biologique, ledit contenant comprenant au moins un moyen de remplissage tel qu'un orifice (ou une ouverture) et au moins un moyen de fermeture permettant de fermer ledit moyen de remplissage, de préférence de manière hermétique,
- au moins un moyen de prélèvement positionné dans l'enceinte dudit contenant, fixe ou mobile, ledit moyen de prélèvement étant adapté pour prélever de l'information biologique et/ou physico-chimique à partir dudit échantillon biologique, ledit moyen de prélèvement étant adapté pour être analysé après prélèvement de ladite information biologique et/ou physico-chimique et/ou placé dans une position de désolidarisation, et
- au moins un conteneur, tel qu'une pochette ou un compartiment, comprenant au moins un agent désinfectant, ledit agent désinfectant étant relargable à l'intérieur du contenant après ouverture dudit conteneur,
- lorsque ledit moyen de prélèvement est adapté pour être analysé après prélèvement de ladite information biologique et/ou physico-chimique, au moins un moyen d'entrave d'analyse distinct dudit moyen de prélèvement, fixe ou mobile, entravant partiellement ou totalement, de préférence totalement, l'analyse dudit moyen de prélèvement,
dans lequel au moins une partie dudit moyen de prélèvement et/ou au moins une partie du moyen d'entrave d'analyse est/sont adapté(s) pour passer d'une première position, dans laquelle l'analyse dudit moyen de prélèvement et/ou la désolidarisation dudit moyen de prélèvement dudit contenant est/sont malaisé(e)(s) ou impossible(s), de préférence impossible(s), vers une deuxième position, dans laquelle l'analyse dudit moyen de prélèvement et/ou la désolidarisation dudit moyen de prélèvement dudit contenant est/sont possible(s), et
dans lequel ladite partie dudit moyen de prélèvement et/ou ladite partie du moyen d'entrave d'analyse est/sont connectée(s) audit conteneur de sorte que l'ouverture dudit conteneur est mécaniquement inductible/déclenchable par le passage de ladite première position vers ladite deuxième position.

Ainsi, ladite partie dudit moyen de prélèvement et/ou ladite partie du moyen d'entrave d'analyse est/sont connectée(s) audit conteneur par une connexion/liaison de telle nature que le passage de ladite première position à ladite deuxième position induit/déclenche l'ouverture dudit conteneur. Ladite connexion/liaison peut être par exemple un fil.

De préférence, ledit conteneur comprend un moyen d'ouverture tel qu'un moyen de rupture, ledit agent désinfectant étant relargable à l'intérieur du contenant après ouverture dudit moyen d'ouverture. Par « moyen d'ouverture », l'on entend tout moyen permettant l'ouverture (par rupture, cassure, déchirure, etc.) dudit conteneur lors du passage de la première position vers la deuxième position. En d'autres termes, ladite partie dudit moyen de prélèvement et/ou ladite partie du moyen d'entrave d'analyse est/sont connectée(s) audit moyen d'ouverture de sorte que l'ouverture dudit moyen d'ouverture est mécaniquement inductible/déclenchable par le passage de ladite première position vers ladite deuxième position.

A titre d'exemple, un tel moyen d'ouverture peut comprendre/être constitué par une zone de moindre résistance ou fragilisée, présente sur ledit conteneur par exemple au niveau de la jonction entre ce dernier et la connexion mécanique (tel qu'un fil), l'ouverture de ce moyen d'ouverture résultant de la force exercée au niveau dudit moyen d'ouverture par ladite connexion lors du passage d'au moins une partie du moyen de prélèvement et/ou d'au moins une partie du moyen d'entrave d'analyse de ladite première position vers ladite deuxième position.

En résumé, le dispositif selon l'invention est adapté pour passer d'une première conformation/configuration dans laquelle l'analyse et/ou la désolidarisation dudit moyen de prélèvement dudit contenant est/sont malaisé(e)(s) ou impossible(s), de préférence impossible(s), vers une deuxième conformation, dans laquelle l'analyse et/ou la désolidarisation dudit moyen de prélèvement dudit contenant est/sont possible(s).

Selon un mode de réalisation préféré, le contenant contient au moins un moyen de culture, tel qu'un milieu de culture, de préférence sous forme déshydratée. Ce ou ces moyen(s) de culture sont importants voire essentiels afin de permettre/favoriser - le cas échéant consécutivement à une étape de réhydratation (dans le cas de moyen(s) de culture déshydraté(s)) - la croissance des micro-organismes cibles. Ceci est particulièrement vrai lorsque l'échantillon biologique d'intérêt est susceptible/suspecté de contenir une quantité minime en micro-organismes cibles.

L'invention concerne également un dispositif adapté pour recevoir au moins un échantillon biologique, ledit dispositif comprenant :
- au moins un contenant, de préférence étanche, adapté pour recevoir ledit échantillon biologique, ledit contenant comprenant au moins un moyen de remplissage tel qu'un orifice et au moins un moyen de fermeture permettant de fermer ledit moyen de remplissage, de préférence de manière hermétique,
- au moins un moyen de prélèvement positionné dans l'enceinte dudit contenant, fixe ou mobile, ledit moyen de prélèvement étant adapté pour prélever de l'information biologique et/ou physico-chimique à partir dudit échantillon biologique, ledit moyen de prélèvement étant adapté pour être analysé après prélèvement de ladite information biologique et/ou physico-chimique, et
- au moins un conteneur, tel qu'une pochette ou un compartiment, comprenant au moins un agent désinfectant, ledit agent désinfectant étant relargable à l'intérieur du contenant après ouverture dudit conteneur,
- au moins un moyen d'entrave d'analyse distinct dudit moyen de prélèvement, fixe ou mobile, entravant partiellement ou totalement, de préférence totalement, l'analyse dudit moyen de prélèvement,
dans lequel au moins une partie dudit moyen de prélèvement et/ou au moins une partie du moyen d'entrave d'analyse est/sont adaptée(s) pour passer d'une première position, dans laquelle l'analyse est malaisée ou impossible (« position d'entrave d'analyse »), de préférence impossible, vers une deuxième position, dans laquelle l'analyse dudit moyen de prélèvement est possible (« position d'analyse »), et
dans lequel ladite partie dudit moyen de prélèvement et/ou ladite partie du moyen d'entrave d'analyse est/sont connectée(s) audit conteneur de sorte que l'ouverture dudit conteneur est mécaniquement inductible par le passage de ladite première position vers ladite deuxième position.

L'invention concerne également un dispositif adapté pour recevoir au moins un échantillon biologique, ledit dispositif comprenant :
- au moins un contenant, de préférence étanche, adapté pour recevoir ledit échantillon biologique, ledit contenant comprenant au moins un moyen de remplissage tel qu'un orifice et au moins un moyen de fermeture permettant de fermer ledit moyen de remplissage, de préférence de manière hermétique,
- au moins un moyen de prélèvement mobile positionné dans l'enceinte dudit contenant, ledit moyen de prélèvement étant adapté pour prélever de l'information biologique et/ou physico-chimique à partir dudit échantillon biologique, ledit moyen de prélèvement étant adapté pour être placé dans une position de désolidarisation, et
- au moins un conteneur, tel qu'une pochette ou un compartiment, comprenant au moins un agent désinfectant, ledit agent désinfectant étant relargable à l'intérieur du contenant après ouverture dudit conteneur,
dans lequel au moins une partie dudit moyen de prélèvement est adaptée pour passer d'une première position, dans laquelle la désolidarisation dudit moyen de prélèvement dudit contenant est malaisée ou impossible, de préférence impossible, vers une deuxième position, dans laquelle la désolidarisation dudit moyen de prélèvement dudit contenant est possible (« position de désolidarisation »), et
dans lequel ladite partie dudit moyen de prélèvement est connectée audit conteneur de sorte que l'ouverture dudit conteneur est mécaniquement inductible par le passage de ladite première position vers ladite deuxième position.

La position de désolidarisation dudit moyen de prélèvement est une position à laquelle ledit moyen de prélèvement fait saillie au moins partiellement à la surface dudit contenant, position dans laquelle ledit moyen de prélèvement est apte à être hermétiquement séparé dudit contenant, par exemple par thermoscellage (thermosoudage) et coupure. Cette position de désolidarisation peut également être dénommée position de transfert, dans la mesure où elle permet le transfert dudit moyen de prélèvement, après désolidarisation de ce dernier, vers un autre contenant par exemple aux fins d'analyse/de confirmation d'analyse.

Par « échantillon biologique », l'on entend, au sens de la présente invention, une quantité définie d'une matière, substance ou produit susceptible/suspecté(e) de contenir de la matière biologique telle que de la matière microbiologique.

Selon la présente invention, l'échantillon biologique peut être de diverses origines, par exemple d'origine alimentaire, environnementale, vétérinaire ou clinique. Parmi les échantillons d'origine alimentaire, l'on peut citer, de façon non-exhaustive, un échantillon de produits lactés (yaourts, fromages...), de viande, de poisson, d'oeuf, de fruit, de légume, d'eau, de boisson (lait, jus de fruits, soda, etc.). Bien évidemment, ces échantillons d'origine alimentaire peuvent aussi provenir de sauces ou de plats plus élaborés ou des matières premières non (ou partiellement) transformées. Un échantillon alimentaire peut également être issu d'une alimentation destinée aux animaux, telle que des tourteaux, des farines animales.

Tel qu'indiqué précédemment, l'échantillon biologique peut être d'origine environnementale et peut consister, par exemple, en un prélèvement de surface, d'eau, d'air, etc.

L'échantillon biologique peut également consister en un échantillon biologique d'origine clinique, pouvant correspondre à des prélèvements de fluide biologique (sang total, sérum, plasma, urine, liquide céphalo-rachidien, etc.), de selles (par exemple diarrhées cholériques), de prélèvements de nez, de gorge, de peau, de plaies, d'organes, de tissus ou de cellules isolées. Cette liste n'est évidemment pas exhaustive.

D'une manière générale, le terme « échantillon » se réfère à une partie ou à une quantité (plus particulièrement une petite partie ou une petite quantité) prélevée à partir d'une ou plusieurs entités aux fins d'analyse. Cet échantillon peut éventuellement avoir subi un traitement préalable, impliquant par exemple des étapes de mélange, de dilution ou encore de broyage, en particulier si l'entité de départ est à l'état solide.

L'échantillon biologique prélevé est, en général, susceptible de - ou suspecté de - contenir au moins un micro-organisme cible. Dans la plupart des cas, ce dernier est un micro-organisme pathogène (tel que Salmonella ou *Vibrio cholerae*) qu'il convient de détecter à des fins sanitaires. Ce micro-organisme cible peut également consister en une bactérie résistante aux antibiotiques (antibio-résistante).

De par sa nature et ses applications, la présente invention est particulièrement adaptée à l'analyse et au transfert aux fins d'analyse d'échantillons biologiques potentiellement à pouvoir pathogène modéré à important (voire même extrêmement important), tels qu'un prélèvement de diarrhée cholérique.

Tel qu'indiqué précédemment, le moyen de fermeture est, de préférence, un moyen de fermeture hermétique visant à obturer le contenant du dispositif selon l'invention. Ce moyen de fermeture peut être réversible ou définitif (irréversible). Ce dernier mode de réalisation est particulièrement avantageux dans la mesure où il permet d'éviter la réouverture (par exemple due à une erreur de l'utilisateur) du moyen de fermeture après introduction de l'échantillon biologique dans le contenant.

A titre de moyen de fermeture réversible, l'on peut citer, par exemple, un bouchon standard ou un moyen de fermeture fixé de manière réversible par collage. Dans ce dernier cas, le passage de la position fermée à la position ouverte dudit moyen de fermeture s'effectue en décollant ce dernier. A contrario, l'opération de fermeture s'effectue par collage dudit moyen de fermeture sur la partie correspondante du contenant comprenant l'ouverture. Selon un mode de réalisation préféré, le moyen de remplissage comprend/est constitué par un appendice creux (par exemple de forme tubulaire), possédant une extrémité libre destinée à accueillir l'échantillon biologique d'intérêt, l'autre extrémité étant connectée audit contenant, de préférence sur un des côtés du contenant, avantageusement constituée de la même matière que ce dernier. De préférence, ladite extrémité libre présente une forme évasée, par exemple une forme d'entonnoir, afin de faciliter la collecte de l'échantillon biologique.

Avantageusement, cet appendice est de forme tubulaire et son diamètre décroît de l'extrémité libre vers celle connectée audit contenant.

Dans ce mode de réalisation préféré, le moyen de fermeture comprend/consiste en une zone thermoscellable/thermosoudable, située au niveau de l'extrémité de l'appendice connecté audit contenant. Dans ce cas, la fermeture dudit appendice s'effectue, de manière définitive, par thermoscellage puis coupure, de préférence à l'aide d'un outil tranchant. Afin d'effectuer ce thermoscellage, l'on utilise de préférence, un dispositif de thermoscellage permettant de thermosceller ladite zone thermoscellable et, de préférence, dans une même action, d'effectuer la coupure de la zone ainsi thermoscellée. De manière alternative, et en particulier lorsque les conditions opérationnelles sont sommaires (par exemple lorsque le dispositif selon l'invention est utilisée dans la brousse, loin de toutes les commodités offertes dans les pays dits « industrialisés »), cette opération de thermoscellage et coupure pourra être réalisée à l'aide d'un briquet et d'une simple paire de ciseaux.

Lorsque l'on utilise un dispositif de thermoscellage, ce dernier possède, avantageusement, un indicateur de température (tel qu'un voyant lumineux) indiquant à l'utilisateur que la température induite par le dispositif de thermoscellage est adéquate.

Par « au moins un moyen de prélèvement d'information biologique et/ou physico-chimique », l'on entend, au sens de la présente invention, tout moyen permettant d'extraire/capturer ladite information biologique et/ou physico-chimique. A titre illustratif, ledit moyen de prélèvement peut être constitué par :
i) un moyen de capture d'information biologique et/ou physico-chimique, par exemple :
   i.1) un moyen de capture sélectif, tel qu'un support de capture fonctionnalisé/sensibilisé par un partenaire de liaison d'un micro-organisme à détecter, ou encore en
   i.2) un moyen de capture non-sélectif, par exemple comprenant/consistant en un moyen d'absorption (par exemple un élément spongieux tel qu'un coton ou un élément compressible tel qu'une éponge), un papier pH ou encore un révélateur de la formation ou de la présence d'un substrat particulier (telle que la formation d'H₂S) destinés à être mis en contact avec ledit échantillon biologique ; ou
ii) un simple réceptacle (par exemple constitué par une membrane flexible repliée) pouvant accueillir en son sein au moins une partie de l'échantillon biologique.

Bien évidemment, le dispositif selon l'invention peut comprendre plusieurs moyens de prélèvement, par exemple une combinaison des susdits moyens de prélèvement i) et ii). A titre illustratif (et non limitatif), ledit dispositif peut comprendre :
- en tant que premier moyen de prélèvement : un réceptacle ii) constitué par une membrane flexible connectée au contenant, ladite membrane flexible étant adaptée pour être invaginée à l'intérieur du contenant dans la susdite première position et au moins partiellement évaginée à l'extérieur du contenant dans la susdite deuxième position, et
- en tant que deuxième moyen de prélèvement : un moyen de capture d'information biologique et/ou physico-chimique i) positionné sur la surface de ladite membrane flexible dans une position adaptée pour être en contact avec l'échantillon biologique dans la susdite première position et n'être plus en contact avec ledit échantillon biologique dans la susdite deuxième position.

La caractéristique « information biologique et/ou physico-chimique » doit être interprétée au sens large dans le contexte de la présente invention comme comprenant une matière biologique en tant que telle, ou au moins l'un de ses constituants (notamment de nature génétique telle que l'ADN, l'ARN ou leurs dérivés ou encore de nature protéique telle que les protéines ou les acides aminés), ainsi que l'information de nature biochimique ou physico-chimique (telle que le pH).

Une matière biologique s'entend de toute matière contenant des informations génétiques et qui est autoreproductible dans un système biologique. A ce titre, l'on peut notamment citer les cellules procaryotes ou eucaryotes, les bactéries ou encore les virus.

La manière dont sont organisés, structurés entre eux les différents éléments du dispositif selon l'invention définit une conformation particulière, à savoir une configuration, un agencement spatial (tridimensionnel) spécifique.

Comme indiqué supra, le conteneur comprenant un agent désinfectant peut consister notamment en une pochette, par exemple placé(e) et fixé(e) à l'intérieur du contenant par une assisse rigide. La paroi de cette pochette ou de ce compartiment est adaptée pour empêcher tout échange du contenu de la pochette ou du compartiment (dans laquelle/lequel se trouve au moins un agent désinfectant) avec l'intérieur du contenant dans ladite première position.

L'agent désinfectant ou les agents désinfectants peut/peuvent être notamment présent(s) sous forme de poudre, de liquide, de pastilles, etc. La nature de ce ou ces agent(s) désinfectant(s) et/ou la quantité dans laquelle ce ou ces derniers sont utilisés est/sont spécifiquement déterminée(s) de telle sorte que l'ensemble des micro-organismes pathogènes pour l'homme et l'animal s'étant développés dans le contenu (par exemple lors d'une phase d'incubation) soient détruits lorsque le ou les agent(s) désinfectant(s) est/sont relargué(s) à l'intérieur du contenant lors du passage de la première position vers la deuxième position.

A titre d'exemple(s), le ou les agent(s) désinfectant(s) est/sont un/des agent(s) antimicrobien(s), tel(s) qu'(e) un/des antibactérien(s), virucide(s), fongicide(s), etc.

Le ou les agent(s) désinfectant(s) peut/peuvent être relargué(s) (libéré(s)) à l'intérieur du contenant après ouverture dudit conteneur. L'ouverture de ce conteneur peut être obtenue par tout moyen approprié. Selon un mode de réalisation préféré, le conteneur est une pochette, laquelle présente une zone de moindre résistance (également dénommée moyen de rupture) permettant son ouverture lors de toute action mécanique réalisée au niveau de ladite zone de moindre résistance. Cette action mécanique peut être de type rupture, déchirement, écrasement, etc.

La connexion entre au moins une partie dudit dispositif distincte dudit moyen de prélèvement et/ou au moins une partie du moyen de prélèvement est une connexion/liaison mécanique (telle qu'un fil) entre :
- au moins une partie du dispositif selon l'invention distinct dudit moyen de prélèvement et/ou au moins une partie dudit moyen de prélèvement, et
- et ledit conteneur.

En d'autres termes, soit au moins une partie du dispositif selon l'invention autre que ledit moyen de prélèvement, soit ledit moyen de prélèvement, soit les deux est/sont mobile(s) de ladite première position vers ladite deuxième position, ce/ces déplacement(s) de ladite première position vers ladite deuxième position générant une force par l'intermédiaire de la connexion/liaison exercée au niveau dudit conteneur, ladite force résultant en l'ouverture de ce dernier. Selon le mode de réalisation préféré dans lequel le conteneur est une pochette présentant une zone de moindre résistance permettant son ouverture lors de toute action mécanique réalisée au niveau de cette zone de moindre résistance, la connexion entre au moins une partie dudit dispositif autre que ledit moyen de prélèvement et/ou au moins une partie dudit moyen de prélèvement et ledit conteneur est réalisée au niveau de la zone de moindre résistance du conteneur, de sorte que le déplacement d'au moins une partie du dispositif autre que le moyen de prélèvement et/ou du moyen de prélèvement de la première position vers la deuxième position induise mécaniquement l'ouverture de cette pochette par exemple par rupture, déchirement, écrasement, etc.

Ledit conteneur peut être placé à l'intérieur ou à l'extérieur du contenant, de préférence à l'intérieur de ce dernier, la condition étant que le ou les agent(s) désinfectant(s) puisse(nt) être relargué(s) à l'intérieur du contenant dans ladite deuxième position. Le conteneur peut consister, par exemple, en un compartiment préformé, réalisé au sein dudit contenant ou en un compartiment distinct, assemblé dans le contenant, par exemple via une soudure à la base dudit compartiment.

Le contenant selon l'invention peut être tout contenant (récipient clos) adapté pour recevoir, analyser et/ou transférer un échantillon biologique. Ce contenant peut être de différent type (sac, tube, poche, etc). Selon un mode de réalisation préféré, le contenant est au moins partiellement constitué en matière flexible. De préférence, cette matière flexible est suffisamment rigide pour que le contenant puisse assurer une tenue suffisante au dispositif selon l'invention, de sorte que ce dernier puisse être stocké (par exemple durant la période d'incubation, si incubation il y a) sans nécessiter l'utilisation d'un portoir. La non-nécessité de recourir à l'utilisation d'un tel portoir est particulièrement intéressante dans le cadre d'une utilisation dudit dispositif hors laboratoire.

Selon un mode de réalisation particulièrement préféré, ledit contenant est constitué au moins partiellement, de préférence en intégralité par un polymère de type polyester tel que le polyéthylène téréphtalate (PET), le polybutylène téréphtalate, le polyéthylène naphtalate (PEN), le polyester saturé étant de préférence le polyéthylène téréphtalate (PET).

De préférence, ledit contenant consiste au moins en partie - et de préférence en totalité - en au moins une couche de papier ou de produit(s) biosourcé(s)) suffisamment épaisse pour assurer une tenue suffisante au dispositif selon l'invention, ou en au moins une couche de carton, ledit papier, produit biosourcé ou carton étant de préférence revêtu sur chacune de ses faces interne et externe d'au moins une couche de polyester saturé, de préférence de PET. A titre illustratif, le polyester saturé, de préférence le PET, est présent dans une concentration allant de 3% à 10% en poids, les 90% à 97% restants étant constitués par ledit papier ou ledit carton. Dans ce mode de réalisation, le contenant peut, le cas échéant, être au moins partiellement déformé afin de limiter l'encombrement du dispositif selon l'invention, sous réserve de respecter l'intégrité de la ou des paroi(s) du contenant. Cette déformation peut, par exemple, consister en un repli en accordéon.

Selon un mode de réalisation préféré, ledit papier ou ledit carton est enduit, sur sa surface interne, par un colorant invisible lorsque ledit colorant n'est pas mouillé (n'est pas en contact avec un élément liquide) mais qui, après contact avec un élément liquide, induit une coloration sur ledit carton ou ledit papier. Ceci permet de détecter aisément une imperfection telle qu'une abrasion ou un trou sur la surface interne et/ou externe dudit carton ou papier constituée par au moins une couche de polymère de type polyester, ladite imperfection étant susceptible d'induire une contamination de l'environnement (dont fait partie l'utilisateur), et/ou de l'échantillon biologique.

L'analyse du moyen de prélèvement d'information biologique et/ou physico-chimique peut être mise en oeuvre à l'aide d'au moins un moyen d'analyse permettant de mesurer directement ou indirectement un ou plusieurs paramètres biologiques et/ou physico-chimiques d'un échantillon biologique (par exemple une variation du pH), de mettre en évidence la présence d'un contaminant ou d'un marqueur particulier dans ledit échantillon. De préférence, un tel moyen d'analyse permet l'analyse directe ou indirecte du ou des micro-organisme(s) cible(s), ainsi que tout ou partie de leurs propriétés et tout changement de milieu généré par ledit ou lesdits micro-organisme(s) tel qu'un changement de couleur de pH par exemple.

Ce moyen de prélèvement de l'information biologique et/ou physico-chimique, lorsqu'il est adapté pour être analyser après mise en contact avec ledit échantillon biologique, peut comprendre un indicateur, marqueur ou autre traceur, lequel pourra être analysé/détecté à l'aide du moyen d'analyse susvisé.

Le moyen de prélèvement d'information biologique et/ou physico-chimique peut également comprendre un moyen de concentration tel qu'un moyen d'immuno-concentration afin d'obtenir une concentration suffisante en micro-organisme(s) recherché(s) au niveau dudit moyen de prélèvement, notamment afin de permettre le transfert du ou des micro-organisme(s) cible(s) vers un autre contenant aux fins d'analyse et/ou de confirmation en séparant, de manière hermétique, ledit moyen de prélèvement dans sa position de désolidarisation.

L'analyse dudit moyen de prélèvement peut être, de manière non-limitative, une analyse visuelle ou optique, de préférence visuelle, une analyse électrique (notamment par mesure d'impédance électrique), une analyse d'ondes (Raman, fluorescence intrinsèque, etc.), une analyse par spectrométrie de masse, et d'une manière générale, pouvant être mise en oeuvre par tout moyen d'analyse permettant d'analyser la susdite information biologique et/ou physico-chimique en vue, par exemple, de vérifier de manière directe ou indirecte la présence de ou des micro-organismes testés au niveau du moyen de prélèvement.

L'analyse visuelle, telle que mentionnée supra, peut être effectuée directement en observant le moyen de prélèvement (par exemple en vue de détecter l'apparition d'une coloration au niveau de ce dernier) ou via un moyen d'amplification optique (permettant par exemple d'augmenter la résolution et/ou la sensibilité de détection) s'interfaçant entre le moyen de prélèvement et l'oeil humain afin de permettre ou, à tout le moins, faciliter la lecture du résultat par l'opérateur. Selon un mode de réalisation préféré, ce moyen d'amplification optique est inclus dans un moyen de télécommunication tel qu'un ordiphone ou téléphone de poche (« smartphone » en langue anglaise). Ainsi le résultat peut être lu :
- par l'opérateur *in situ* directement par lecture visuelle du moyen de prélèvement et/ou
- par un analyste *ex situ* (à savoir localisé à distance du site de prélèvement, par exemple à plusieurs centaines voire milliers de kilomètres de celui-ci).

Selon un mode de réalisation particulier, le résultat est lu à la fois par l'opérateur *in situ* et par l'analyste *ex situ,* ce dernier confirmant ou infirmant la primo-analyse réalisée par l'opérateur.

Selon un mode de réalisation préféré, ladite partie du moyen d'entrave d'analyse est adaptée pour coopérer avec ladite partie du moyen de prélèvement dans ladite première position de façon à rendre l'analyse dudit moyen de prélèvement au sein du contenant malaisée ou impossible, de préférence impossible, dans ladite première position, ladite partie du moyen d'entrave d'analyse étant adaptée pour être dégagée partiellement ou totalement, de préférence totalement, de ladite partie du moyen de prélèvement et/ou inversement dans ladite deuxième position, de façon à permettre l'analyse dudit au moins un moyen de prélèvement sans sortir ce dernier dudit dispositif.

En d'autres termes, au moins une partie dudit moyen de prélèvement et/ou dudit moyen d'entrave d'analyse est/sont connecté(s) audit conteneur, le passage de ladite première position vers ladite deuxième position consistant en un dégagement partiel ou total, de préférence total, dudit moyen d'analyse dudit moyen d'entrave d'analyse et/ou inversement.

Le moyen d'entrave d'analyse est sélectionné en fonction du type d'analyse utilisé et de la nature du moyen de prélèvement d'information biologique et/ou physico-chimique à analyser. Au titre de ce moyen d'entrave d'analyse, tout moyen peut être utilisé à la condition que celui-ci entrave au moins partiellement - et de préférence totalement - l'analyse dudit moyen d'analyse dans ladite première position. A titre illustratif, lorsque l'analyse est de type électromagnétique, le moyen d'entrave d'analyse peut être constitué d'une cage de Faraday, positionnée de telle sorte que l'analyse électromagnétique du moyen de prélèvement dans ladite première position est partiellement ou totalement (de préférence totalement) entravée. Cette analyse n'est possible qu'après retrait de cette cage de Faraday, lequel retrait induit mécaniquement l'ouverture du conteneur comprenant au moins un agent désinfectant. A titre de cage de Faraday, l'on utilise, par exemple, une enveloppe métallique (par exemple de forme tubulaire).

Par dégagement partiel ou total, de préférence total, dudit moyen d'entrave d'analyse dudit moyen de prélèvement et/ou inversement, l'on entend, au sens de la présente invention :
- un retrait du moyen d'entrave d'analyse, se traduisant par un déplacement dudit moyen d'entrave d'analyse de ladite première position vers ladite deuxième position; et/ou
- un déplacement dudit moyen de prélèvement d'information biologique et/ou physico-chimique de ladite première position vers ladite deuxième position;
- ce ou ces déplacement(s) étant suffisant(s) pour permettre l'analyse dudit moyen de prélèvement dans ladite deuxième position.

En d'autres termes, afin d'être en mesure d'analyser ledit moyen de prélèvement, l'on procède soit au retrait dudit moyen d'entrave d'analyse, par exemple en exerçant une force telle qu'une force de traction ou de rotation sur ce dernier, soit l'on effectue le déplacement dudit moyen de prélèvement, par exemple en exerçant une force telle qu'une force de traction ou de rotation sur ce dernier, soit les deux.

Ainsi, l'opérateur, désireux d'effectuer l'analyse dudit moyen de prélèvement, par toute technique et tout moyen d'analyse appropriés, est contraint d'exercer une force de façon à dégager ledit moyen d'entrave d'analyse dudit moyen de prélèvement et/ou inversement, ladite force, étant répercutée selon un mode de réalisation particulier, par la connexion/liaison mécanique entre au moins une partie du moyen de prélèvement et/ou du moyen d'entrave d'analyse et le conteneur, induisant ainsi l'ouverture de ce dernier, par exemple par rupture ou par déchirure, de préférence au niveau de la zone du conteneur de moindre résistance/fragilisée.

Avantageusement, ledit moyen de prélèvement est adapté pour être analysé dans ladite deuxième position, ledit moyen de prélèvement comprenant au moins un moyen de capture d'information biologique et/ou physico-chimique adapté pour être mis en contact avec ledit échantillon biologique dans ladite première position et analysé dans ladite deuxième position.

Ce moyen de capture d'information biologique et/ou physico-chimique peut consister en :
- un moyen de capture sélectif, tel qu'un support de capture fonctionnalisé/sensibilisé par un partenaire de liaison d'un micro-organisme à détecter, ou encore en
- un moyen de capture non-sélectif, par exemple comprenant/consistant en un moyen d'absorption (par exemple un élément spongieux tel qu'un coton ou un élément compressible tel qu'une éponge) ou encore un papier pH destinés à être mis en contact avec ledit échantillon biologique.

Selon un mode de réalisation particulièrement préféré, ledit moyen de capture est adapté pour être analysé, dans ladite deuxième position, par lecture visuelle ou optique, de préférence par lecture visuelle, ledit moyen d'entrave d'analyse étant un élément entravant au moins partiellement, de préférence totalement, la transmission de l'information lumineuse entre ledit moyen de prélèvement et l'oeil humain ou ledit moyen de lecture optique dans ladite première position.

Dans le cadre d'une lecture optique, cette dernière peut être effectuée via au moins un moyen de lecture optique positionné à l'extérieur du contenant tel qu'une caméra ou un spectroscope de Raman ou tout système autorisant ladite lecture optique, notamment en vue de détecter, de manière directe ou indirecte, la présence du ou des micro-organisme(s) recherché(s) au niveau du moyen de prélèvement.

Selon ce mode de réalisation préférée, ledit moyen de prélèvement d'information biologique et/ou physico-chimique comprend un moyen susceptible d'émettre un signal lumineux dans le spectre visible, infrarouge ou ultraviolet, de préférence dans le spectre visible (par exemple coloration ou fluorescence) afin que ce signal lumineux puisse être détecté par lecture visuelle ou optique, de préférence par lecture visuelle.

Tel qu'indiqué précédemment, l'on utilise, aux fins de détection optique, un moyen de lecture optique positionné à l'extérieur du contenant tel qu'une caméra ou spectroscope de Raman. De manière avantageuse, l'on utilise un moyen de lecture optique adapté pour être utilisé sur le terrain, à savoir hors laboratoire, tel qu'un spectroscope de Raman portatif.

En fonction du positionnement de la zone de lecture, la lecture visuelle ou optique peut être effectuée :
- soit lorsque ledit moyen de capture n'est plus en contact avec l'échantillon biologique (et éventuellement le milieu de culture), ou, a contrario,
- soit lorsque ledit moyen de capture est toujours en contact avec ledit échantillon biologique (et éventuellement le milieu de culture).

Selon un mode de réalisation préféré, l'élément entravant au moins partiellement, de préférence totalement, la transmission de l'information lumineuse entre ledit moyen de prélèvement (en l'espèce ledit moyen de capture) et l'oeil humain ou ledit moyen de lecture optique dans ladite première position consiste en au moins une zone opaque au niveau d'au moins une paroi dudit contenant et/ou consiste en un cache mobile, de préférence positionné à l'intérieur dudit contenant.

Ainsi, le dispositif selon l'invention comprend au moins un contenant (par exemple un sac), lequel peut présenter sur au moins l'une de ses parois, une zone opaque entravant partiellement ou totalement, de préférence totalement, la lecture visuelle ou optique dudit moyen de prélèvement dans ladite première position. Dans cette première configuration, l'opérateur ne peut pas effectuer la lecture visuelle ou optique du moyen de prélèvement d'information biologique et/ou physico-chimique. Cet opérateur, souhaitant réaliser la lecture visuelle ou optique dudit moyen de prélèvement, est contraint d'exercer une force, par exemple de type traction ou rotation, sur ledit moyen de prélèvement afin d'amener ce dernier au niveau d'une zone du dispositif possédant au moins une paroi translucide ou transparente (« zone de lecture »), de préférence transparente, afin de pouvoir réaliser la lecture visuelle ou optique dudit moyen de prélèvement et ainsi obtenir le résultat recherché. Selon un mode de réalisation préféré, la force exercée sur ledit moyen de prélèvement afin d'obtenir le déplacement de ce dernier vers ladite zone de lecture est répercutée mécaniquement au niveau du conteneur, via la liaison entre ce dernier et le moyen de prélèvement, afin d'obtenir l'ouverture dudit conteneur et le relargage dudit au moins un agent désinfectant à l'intérieur du conteneur.

Selon un mode de réalisation particulièrement préféré, l'ensemble des parois du contenant sont opaques, empêchant ainsi l'analyse visuelle ou optique du moyen de prélèvement (en l'espèce dudit moyen de capture d'information biologique et/ou physico-chimique). La zone de lecture se trouve alors à l'extérieur dudit contenant mais toujours dans l'enceinte du dispositif selon la présente invention, par exemple dans une zone extérieure audit contenant mais connectée à ce dernier via une membrane flexible (dans ce cas la zone de lecture est localisée entre la membrane flexible et la paroi dudit contenant à laquelle ladite membrane flexible est connectée).

Concernant l'utilisation d'un cache mobile tel qu'une enveloppe opaque amovible enveloppant au moins partiellement ledit moyen de prélèvement, l'on exerce une force, par exemple de type traction ou rotation, sur ledit cache mobile afin d'obtenir son retrait total ou partiel, de préférence total, du moyen de prélèvement d'information biologique et/ou physico-chimique, afin de rendre ledit moyen de prélèvement disponible pour une lecture visuelle ou optique, de préférence optique. Dans ce mode de réalisation, le moyen de prélèvement est de préférence fixe et le cache mobile est adapté pour se déplacer d'une première position entravant la lecture visuelle ou optique vers une deuxième position permettant cette dernière. Selon un mode de réalisation particulier, à la fois le moyen de prélèvement et le cache peuvent également être mobiles d'une première position vers une deuxième position.

Avantageusement, ledit moyen de capture d'information biologique et/ou physico-chimique consiste en un moyen de capture de micro-organisme(s) tel qu'un support de capture apte à capturer le ou les micro-organisme(s) recherché(s), ledit moyen de capture de micro-organismes étant apte à être analysé, après capture du ou des micro-organisme(s) recherché(s), par au moins un moyen d'analyse.

La connexion/liaison (tel qu'un fil) entre le conteneur et ledit moyen de prélèvement et/ou ledit moyen d'entrave d'analyse est configurée de telle manière que la désinfection, induite par le passage de ladite première position vers ladite deuxième position, se produit :
- lorsque ledit moyen de capture n'est plus en contact avec l'échantillon biologique (et éventuellement le milieu de culture), ou, a contrario,
- lorsque ledit moyen de capture est toujours en contact avec ledit échantillon biologique (et éventuellement le milieu de culture).

Bien que ces deux options soient comprises dans la portée de la présente invention, le premier scénario est particulièrement avantageux, dans la mesure où il permet de préserver l'intégrité du ou des micro-organisme(s) recherché(s), ce qui peut s'avérer important dans l'optique d'une (re)mise en culture de ce/ces dernier(s) aux fins de confirmation d'analyse.

A noter que, même dans le deuxième scénario, une confirmation d'analyse peut être réalisée sur des micro-organismes morts, notamment en utilisant les techniques de biologie moléculaire/d'identification génétique (telles que la technique « PCR »).

Un exemple de moyen de capture de micro-organisme(s) utilisable selon la présente invention consiste en un support de capture fonctionnalisé/sensibilisé par un partenaire de liaison spécifique ou non spécifique (de préférence spécifique) d'au moins un micro-organisme cible. Selon un mode de réalisation préféré, le partenaire de liaison spécifique est sélectionné dans le groupe comprenant les anticorps, les fragments Fab, les fragments Fab', les protéines de phages recombinantes ou non, les phages, les aptamers ou tout autre ligand plus ou moins spécifiques bien connus de l'homme du métier.

La révélation de la présence des micro-organismes cibles au niveau du support de capture peut être effectuée par l'intermédiaire de tout système de révélation approprié, c'est-à-dire apte à permettre la détection du ou des micro-organisme(s) cible(s). Par « système de révélation », l'on entend toute molécule capable de se coupler avec les micro-organismes ou les partenaires de liaison desdits micro-organismes et permettant, par leurs propriétés de transduction (fluorescence, coloration, radioactivité, etc.) de révéler la présence desdits micro-organismes. Cette révélation de la présence des micro-organismes cibles peut être notamment obtenue par visualisation ou lecture optique, tel qu'indiqué précédemment, d'une coloration (telle qu'une coloration rouge due à la réduction chlorure de 2,3,5-triphényle tétrazolium, dont l'acronyme est « TTC », par les micro-organismes recherchés) ou d'une fluorescence sur tout ou partie du support de capture.

Selon un mode de réalisation préféré, le système de révélation est basé sur la réduction de certains sels de tétrazolium par les micro-organismes, en particulier du chlorure de 2,3,5-triphényle tétrazolium par lesdits micro-organismes. Simultanément à la croissance, le TTC (incolore sous sa forme non réduite) est internalisé par lesdits micro-organismes, puis réduit par ces derniers en triphényl-formazan (de couleur rouge), colorant ainsi les micro-organismes présents sur le support de capture en rouge, cette coloration pouvant ainsi être détectée par lecture visuelle ou optique dans ladite deuxième position.

A titre de support de capture, peuvent être notamment cités les supports particulaires, éventuellement magnétiques ou encore les supports monobloc, éventuellement poreux. Le support de capture peut être simplement un support inerte, tel qu'une plaque de matériau plastique ou de la fibre de verre, ou, avantageusement, peut être sensibilisé avec un partenaire de liaison éventuellement spécifique. Le support de capture peut également consister en un support monobloc compressible. Selon un mode de réalisation particulier, le support de capture peut ne faire qu'un avec le moyen de détection. C'est le cas, par exemple, quand le support de capture est constitué par un biocapteur électrochimique ou une fibre optique.

Concernant le partenaire de liaison, lorsqu'un tel partenaire de liaison est fixé sur un support de capture, celui-ci est avantageusement sélectionné parmi les anticorps, les fragments Fab, les fragments Fab', les protéines de phages recombinantes ou non, les phages, les lectines, les acides nucléiques de type aptamère ou tout autre ligand bien connu de l'homme du métier.

Selon un mode de réalisation particulièrement préféré, le moyen de prélèvement est adapté pour être placé dans une position de désolidarisation/séparation, ledit moyen consistant en une membrane flexible connectée audit contenant, ladite membrane flexible étant adaptée pour être invaginée à l'intérieur du contenant dans ladite première position et au moins partiellement évaginée à l'extérieur du contenant dans ladite deuxième position (« position de désolidarisation»).

Cette membrane flexible consiste, au sens de la présente invention, en une membrane/paroi souple et hermétique afin d'éviter les risques de contamination.

La première position (invaginée) est une position dans laquelle ladite membrane flexible a pénétré par retournement en doigt de gant à l'intérieur dudit contenant (forme en doigt de gant retourné).

Le passage de la position invaginée à la position évaginée s'effectue par retournement comme un doigt de gant de ladite membrane flexible, laquelle, en position évaginée, fait saillie à la surface dudit contenant dans une forme de doigt de gant.

Cette forme en doigt de gant retourné, invaginé à l'intérieur du contenant permet de protéger l'intégrité de ladite membrane flexible dans ladite première position (qui peut être dénommée « position de protection »), qui représente la position dans laquelle le dispositif selon l'invention est stocké et/ou transporté. Cette forme en doigt de gant retourné du moyen de prélèvement permet donc de limiter les risques de déchirure, rupture ou autres accrocs dans la membrane flexible constituant le moyen de prélèvement lors du stockage du dispositif selon l'invention et du transport de ce dernier.

Selon ce mode de réalisation, ledit moyen de prélèvement peut être « passif », à savoir qu'il ne comprend pas de moyen de capture d'information biologique et/ou physico-chimique mais permet le prélèvement aux fins de transfert en vue d'analyse(s) ultérieure(s) d'un aliquote de l'échantillon biologique d'intérêt. Bien évidemment, dans sa position invaginée (« première position » ou « position de protection »), le moyen de prélèvement ne permet pas d'effectuer un tel prélèvement. Ce dernier est possible dans la deuxième position (« position de désolidarisation » ou « position de transfert », à savoir quand ledit moyen de prélèvement est au moins partiellement évaginé (à savoir qu'il fait au moins partiellement saillie à la surface dudit contenant).

Le passage dudit moyen de prélèvement de la première position (invaginée) vers la position de désolidarisation (au moins partiellement évaginée) peut être effectué, par exemple, en appliquant une force de traction sur un moyen de préhension connecté à la surface externe de ladite membrane flexible ou, de manière préférée, en générant une surpression d'air (par exemple par compression d'au moins une paroi flexible dudit contenant) à l'intérieur dudit contenant, de façon à déclencher l'« évagination » dudit moyen de prélèvement, à savoir le retournement en doigt de gant de ladite membrane flexible. Bien évidemment, et en particulier dans le mode de réalisation selon lequel ladite au moins une paroi flexible est adaptée pour être comprimée afin d'induire l'évagination du moyen de prélèvement par surpression d'air, les paroi(s) et membrane flexibles, ainsi que, plus généralement, les différents constituants du dispositif selon l'invention, sont soigneusement sélectionnés pour que leur intégrité soit préservée lors du passage de la position invaginée vers la position évaginée. Ceci est important afin de garantir la sécurité sanitaire de l'opérateur et de son environnement.

Dans la deuxième position (« position de désolidarisation »), l'aliquote de l'échantillon biologique d'intérêt peut être introduit dans le doigt de gant formé par le moyen de prélèvement évaginé par tout moyen, par exemple simplement en inclinant suffisamment le dispositif selon l'invention pour qu'une partie de l'échantillon biologique puisse couler dans le moyen de prélèvement évaginé. Une alternative afin d'introduire l'aliquote désiré au sein du moyen de prélèvement dans ladite position de désolidarisation consiste, par exemple, à comprimer le contenant avec une intensité suffisamment importante pour qu'une partie de l'échantillon biologique (et éventuellement du milieu de culture) puisse être transférée dudit contenant vers le moyen de prélèvement en position de désolidarisation (en forme de doigt de gant comme indiqué précédemment). Dans ce dernier cas, il est nécessaire qu'au moins une paroi dudit contenant soit déformable (par exemple soit réalisée au moins partiellement en matière souple ou flexible), de manière à ce que la compression appliquée au niveau de cette/ces paroi(s) déformable(s) puisse déformer cette dernière et ainsi induire le transfert d'au moins une partie de l'échantillon biologique (et éventuellement du milieu de culture) via une élévation du niveau de ce dernier au sein dudit contenant sans danger pour l'opérateur et l'environnement.

A des fins de clarté, il convient de noter que, dans ce mode de réalisation, au moins une partie dudit moyen de prélèvement est connectée audit conteneur par une connexion/liaison mécanique adaptée pour induire l'ouverture dudit conteneur lorsque ledit moyen de prélèvement se déplace de sa première position (position invaginée) vers sa position de désolidarisation (position évaginée).

En conséquence, le simple fait d'effectuer cette procédure de transfert d'échantillon biologique du contenant vers ledit moyen de prélèvement en position évaginée (deuxième position) va induire *ipso facto* la désinfection de la partie de l'échantillon biologique qui demeure au sein dudit contenant après ladite procédure de transfert. Ainsi, et sans action spécifique de l'opérateur visant à induire la désinfection du contenant, l'on obtient de manière automatique/systématique la désinfection de la matière biologique restante au sein dudit contenant.

Selon un mode de réalisation avantageux, ladite membrane flexible comprend au moins un moyen de capture d'information biologique et/ou physico-chimique, ledit moyen de capture d'information biologique et/ou physico-chimique étant positionné sur la surface de ladite membrane flexible dans une position adaptée pour être en contact avec l'échantillon biologique dans ladite première position et n'être plus en contact avec ledit échantillon biologique dans ladite deuxième position.

Le positionnement dudit moyen de capture d'information biologique et/ou physico-chimique sur la surface de ladite membrane flexible est défini pour que ce moyen de capture soit en contact avec l'échantillon biologique lorsque ledit moyen de prélèvement est en position invaginée dans le contenant, à savoir présente une forme de doigt de gant retourné, et pour n'être plus en contact avec ledit échantillon biologique en position au moins partiellement évaginée à l'extérieur du contenant, à savoir en forme de doigt de gant. Si l'on poursuit l'analogie avec une forme en doigt de gant retourné/doigt de gant, le moyen de capture d'information biologique et/ou physico-chimique est de préférence positionné au niveau de l'extrémité du doigt de gant retourné en contact avec l'échantillon biologique en première position (invaginée). Ce mode de réalisation est particulièrement préféré puisqu'il offre la possibilité, outre le fait de pouvoir éventuellement prélever un aliquote de l'échantillon biologique d'intérêt aux fins de transfert comme décrit précédemment, d'effectuer la capture d'au moins une information biologique et/ou physico-chimique d'intérêt en première position (position invaginée), en laissant ledit moyen de capture en contact avec l'échantillon biologique d'intérêt durant un laps de temps suffisant pour effectuer la capture de la ou des informations biologique(s) et/ou physico-chimique(s) d'intérêt, puis, dans un second temps (lorsque l'opérateur estime que le moyen de capture est resté en contact avec l'échantillon biologique durant un laps de temps suffisant), d'effectuer le transfert dudit moyen de capture en exerçant une force, par exemple de type traction ou rotation, sur ledit moyen de prélèvement permettant d'obtenir son déplacement de sa première position (invaginée) vers sa position de désolidarisation (évaginée). Une fois la position de désolidarisation atteinte, l'opérateur peut, s'il le désire, séparer (désolidariser) hermétiquement le moyen de prélèvement contenant ledit moyen de capture d'information biologique et/ou physico-chimique (ainsi qu'éventuellement un aliquote d'échantillon biologique) afin d'envoyer ledit moyen de prélèvement séparé du contenant à un laboratoire, en vue, par exemple, d'effectuer l'analyse et/ou la confirmation d'analyse dudit moyen de capture « emprisonné » à l'intérieur du moyen de prélèvement scellé par thermoscellage. Tel qu'indiqué précédemment, et si cela est conforme au souhait de l'opérateur, outre le moyen de capture d'information biologique et/ou physico-chimique, le moyen de prélèvement hermétiquement séparé dudit contenant peut comprendre un aliquote issu de l'échantillon biologique initialement introduit au sein du contenant. Ceci peut notamment s'avérer intéressant si l'on souhaite (re)mettre en culture tout ou partie de la matière biologique, en particulier la matière biologique, se trouvant dans cet aliquote.

Selon un mode de réalisation particulier, avant séparation hermétique du moyen de prélèvement, ce dernier peut être analysé dans ladite position de désolidarisation. Dans cette configuration, le moyen de capture d'information biologique et/ou physico-chimique est, de préférence, un moyen de capture de micro-organisme(s) tel que défini précédemment, pouvant avantageusement être analysé par lecture visuelle ou optique dans ladite position de désolidarisation ; la membrane flexible constituant le moyen de prélèvement étant, dans ce mode de réalisation, translucide ou transparente, de préférence transparente, afin de permettre la lecture visuelle ou optique du moyen de capture de micro-organisme(s). Avantageusement, les parois du contenant sont opaques de sorte que cette analyse par lecture visuelle ou optique ne puisse pas être effectuée au sein du contenant mais requière le déplacement dudit moyen de prélèvement, comprenant le moyen de capture de micro-organisme(s), d'une première position dans laquelle l'analyse dudit moyen de capture de micro-organisme(s) est impossible vers ladite deuxième position correspondant, dans ce mode de réalisation, à la fois à la position d'analyse et à celle de désolidarisation, et dans laquelle l'analyse par lecture visuelle ou optique est possible, en préalable à la désolidarisation dudit moyen de prélèvement. Les parois opaques du contenant correspondent, dans ce mode de réalisation, au susdit moyen d'entrave d'analyse. Ce dernier est, toujours dans ce mode de réalisation, fixe, alors que le moyen de prélèvement est adapté pour passer d'une première position dans laquelle la lecture visuelle ou optique est impossible vers une deuxième position dans laquelle celle-ci est possible.

Ainsi, le dispositif selon la présente invention offre une latitude d'utilisation importante à l'opérateur. En effet, ce dernier peut, s'il le désire :
- utiliser simplement ledit dispositif pour transférer un aliquote de l'échantillon biologique d'intérêt vers un autre contenant afin, par exemple, d'effectuer des analyses plus approfondies ;
- transférer au moins un moyen de capture d'information biologique et/ou physico-chimique ayant été préalablement mis en contact avec l'échantillon biologique à analyser vers un autre contenant afin d'effectuer une analyse et/ou confirmation d'analyse additionnelle(s) ;
- effectuer l'analyse, de préférence par lecture visuelle ou optique, dudit moyen de capture d'information biologique et/ou physico-chimique présent sur ledit moyen de prélèvement, dans la position de désolidarisation, éventuellement en préalable à un tel transfert, etc.

La forme préférentielle du moyen de prélèvement selon l'invention dans sa première position (position invaginée) à savoir une forme en doigt de gant retourné dont au moins l'extrémité est en contact avec l'échantillon biologique d'intérêt dans ladite première position est particulièrement avantageuse, lorsqu'au moins un moyen de capture d'information biologique et/ou physico-chimique est positionné à cette extrémité pour être en contact avec l'échantillon biologique dans la position du départ puisque l'opérateur peut glisser son doigt à l'intérieur du moyen de prélèvement en forme de doigt de gant retourné afin de déplacer le moyen de capture d'information biologique et/ou physico-chimique vers une ou plusieurs zones d'intérêt. Ceci peut présenter un avantage notamment si l'échantillon biologique est sous forme semi-solide (tel qu'un échantillon de selles) ou si l'échantillon n'est pas correctement/suffisamment homogénéisé. Ainsi, l'opérateur peut manuellement diriger le moyen de capture d'information biologique et/ou physico-chimique vers/dans les zones de l'échantillon biologique ou l'on peut légitimement s'attendre à ce que l'information biologique et/ou physico-chimique recherchée soit présente en quantité plus importante.

Selon un mode de réalisation préféré, le conteneur comprend également au moins un marqueur tel qu'un marqueur coloré permettant de vérifier le relargage dudit agent désinfectant à l'intérieur du contenant après ouverture dudit conteneur.

Un tel marqueur coloré peut consister, à titre illustratif, en un indicateur coloré, dont la couleur est susceptible d'être modifiée sous l'effet d'une variation de pH.

De préférence, le ou les agents désinfectants sont présents en excès au sein du conteneur afin d'assurer une désinfection suffisante de l'intérieur du contenant même dans l'éventualité d'un relargage partiel.

L'apparition d'une coloration liée audit marqueur coloré dans le contenu dudit contenant (comprenant notamment l'échantillon biologique) témoigne de l'ouverture dudit conteneur et/ou de la désinfection effective de tout ou partie de l'intérieur du contenant après que l'opérateur s'est livré à une opération d'analyse et/ou de transfert d'échantillon biologique. Cette désinfection effective résulte de la répartition homogène du désinfectant à l'intérieur du contenant et de la destruction microbienne en son sein.

Selon un mode de réalisation préféré, ledit moyen de prélèvement est connecté audit contenant par un moyen d'inviolabilité, tel qu'une bague d'inviolabilité.

Selon un mode de réalisation préféré, le dispositif selon l'invention comprend une pluralité de moyens de prélèvement identiques ou différents positionnés dans l'enceinte dudit contenant, les moyens de prélèvement étant connectés ensemble de telle manière que le passage de ladite première position vers ladite deuxième position d'au moins un des moyens de prélèvement provoque, dans le même temps, le passage de ladite première position vers ladite deuxième position des autres moyens de prélèvement, induisant ainsi l'ouverture dudit conteneur.

Ce mode de réalisation s'avère particulièrement avantageux, dans la mesure où il permet de détecter/mesurer plusieurs informations biologiques et/ou physico-chimiques différentes, en utilisant différents moyens de prélèvement d'information biologique et/ou physico-chimique. Ainsi, l'opérateur peut effectuer, de manière quasi-concomitante, la détection d'une information biologique, comme par exemple la détection d'un micro-organisme cible, et la détection d'un paramètre physico-chimique, comme par exemple la mesure d'un pH. Bien évidemment, le nombre et le type de moyens de prélèvement utilisés dépendent du nombre et de la nature des informations biologiques et/ou physico-chimiques recherchées.

Selon un mode de réalisation particulier, le dispositif selon l'invention peut comprendre une pluralité de compartiments (de préférence étanches et indépendants les uns des autres, éventuellement inclus dans un contenant commun) adaptés pour recevoir au moins un échantillon biologique, chacun des compartiments étant avantageusement relié à un moyen de remplissage commun (représentant ainsi une entrée commune), chacun desdits compartiments comprenant, en outre :
- au moins un moyen de prélèvement positionné dans l'enceinte dudit compartiment, fixe ou mobile, ledit moyen de prélèvement étant adapté pour prélever de l'information biologique et/ou physico-chimique à partir dudit échantillon biologique, ledit moyen de prélèvement étant adapté pour être analysé après prélèvement de ladite information biologique et/ou physico-chimique et/ou placé dans une position de désolidarisation, et
- au moins un conteneur, tel qu'une pochette ou un sous-compartiment, comprenant au moins un agent désinfectant, ledit agent désinfectant étant relargable à l'intérieur du compartiment après ouverture dudit conteneur,
- lorsque ledit moyen de prélèvement est adapté pour être analysé après prélèvement de ladite information biologique et/ou physico-chimique, au moins un moyen d'entrave d'analyse distinct dudit moyen de prélèvement, fixe ou mobile, entravant partiellement ou totalement, de préférence totalement, l'analyse dudit moyen de prélèvement,
dans lequel au moins une partie dudit moyen de prélèvement et/ou au moins une partie du moyen d'entrave d'analyse est/sont adaptée(s) pour passer d'une première position, à l'intérieur dudit compartiment, dans laquelle l'analyse dudit moyen de prélèvement et/ou la désolidarisation dudit moyen de prélèvement dudit compartiment (ou du contenant commun) est/sont malaisé(e)(s) ou impossible(s), de préférence impossible(s), vers une deuxième position, dans laquelle l'analyse dudit moyen de prélèvement et/ou la désolidarisation dudit moyen de prélèvement dudit compartiment (ou du contenant commun) est/sont possible(s), et
dans lequel ladite partie dudit moyen de prélèvement et/ou ladite partie du moyen d'entrave d'analyse est/sont connectée(s) audit conteneur de sorte que l'ouverture dudit conteneur est mécaniquement inductible par le passage de ladite première position vers ladite deuxième position.

Avantageusement, et toujours dans ce mode de réalisation particulier, le moyen de prélèvement selon la présente invention est adapté pour être mis en contact avec l'échantillon biologique dans une première position puis analysé sans être sorti dudit dispositif dans une deuxième position et/ou déplacé d'une première position localisée à l'intérieur dudit compartiment (« position de protection »), vers une position de désolidarisation (également dénommée « position de transfert »).

Chacun des compartiments est donc indépendant des autres compartiments. A titre illustratif, l'on peut exercer une force sur le moyen de prélèvement numéro 1 du compartiment numéro 1, afin d'effectuer l'analyse et/ou le transfert dudit moyen de prélèvement numéro 1. Ceci induit mécaniquement (et automatiquement) la désinfection du compartiment numéro 1, par le mécanisme exposé dans la présente demande. En revanche, les autres compartiments (compartiments numéro 2, numéro 3, numéro 4, etc.) ne sont pas affectés par la désinfection induite du compartiment numéro 1 et l'analyse et/ou le transfert d'échantillon biologique dans ces autres compartiments pourra/pourront être effectué(s) ultérieurement, la matière biologique n'ayant pas été détruite par l'agent désinfectant déversé au sein du compartiment numéro 1.

Un autre objet de l'invention concerne un procédé permettant d'assurer la désinfection de tout ou partie de l'intérieur d'un contenant adapté pour recevoir au moins un échantillon biologique, ledit procédé mettant en oeuvre le dispositif selon l'invention, ledit procédé comprenant les étapes suivantes :
a) introduire, par ledit moyen de remplissage, au moins un échantillon biologique au sein dudit contenant,
b) fermer ledit moyen de remplissage, de préférence de manière hermétique, à l'aide dudit moyen de fermeture,
c) exercer une force, par exemple de type traction ou rotation, sur au moins une partie dudit moyen de prélèvement et/ou au moins une partie dudit moyen d'entrave d'analyse afin de passer de ladite première position vers ladite deuxième position, en vue d'analyser et/ou de désolidariser ledit moyen de prélèvement,
d) effectuer, dans ladite deuxième position, l'analyse dudit moyen de prélèvement à l'aide d'au moins un moyen d'analyse positionné à l'extérieur dudit conteneur, et/ou
d') désolidariser, dans ladite deuxième position (position de désolidarisation), ledit moyen de prélèvement, de préférence par séparation hermétique, par exemple par thermoscellage et coupure, en vue d'analyser tout ou partie dudit échantillon biologique présent au niveau dudit moyen de prélèvement lors d'une étape d'analyse e) réalisée dans un autre contenant,
le passage de ladite première position vers ladite deuxième position induisant mécaniquement l'ouverture dudit conteneur, permettant ainsi d'assurer la désinfection de tout ou partie dudit contenant.

Les différents éléments du dispositif selon l'invention sont tels que décrits précédemment.

L'expression « moyen d'analyse positionné à l'extérieur dudit contenant » doit être interprétée au sens large. En effet, un tel moyen d'analyse, par exemple dans le cas d'une analyse de type visuel ou optique, peut notamment consister en l'oeil humain ou encore tout moyen de lecture optique.

Tel qu'indiqué précédemment, à l'étape d'), la séparation dudit moyen de prélèvement est de préférence réalisée par thermoscellage et coupure. Ces deux opérations peuvent être réalisées via deux manipulations distinctes, par exemple une première opération de thermoscellage puis une deuxième opération de coupure de préférence avec un objet tranchant ou effectuées via une seule et même manipulation, ladite manipulation comprenant le thermoscellage puis, dans la continuité de ce dernier, la coupure. Lorsque l'environnement dans lequel l'opérateur se trouve n'offre pas un accès aisé aux dispositifs de thermoscellages élaborés (« thermoscelleurs ou thermosoudeurs ») permettant d'effectuer ce thermoscellage en une seule manipulation, l'opérateur peut se contenter d'utiliser un simple briquet afin de réaliser l'opération de thermoscellage puis effectuer la coupure à l'aide d'une paire de ciseaux.

L'analyse e) réalisée dans un autre contenant peut être réalisée dans un dispositif d'analyse indépendant, par exemple un dispositif d'analyse automatisé de type VIDAS®.

Avantageusement, à l'étape c), l'on exerce une force de traction sur au moins un moyen de préhension connecté à ladite partie dudit moyen de prélèvement et/ou à ladite partie dudit moyen d'entrave d'analyse.

Par moyen de préhension, l'on entend, au sens de la présente invention, tout moyen permettant de prendre, saisir au moins une partie du dispositif distincte dudit moyen de prélèvement et/ou au moins une partie dudit moyen de prélèvement. Un tel moyen de préhension peut consister, en une poignée, une languette, une tirette, etc.

En d'autres termes, soit ladite partie du dispositif distincte dudit moyen de prélèvement (par exemple consistant en le moyen d'entrave d'analyse tel qu'indiqué précédemment), soit le moyen de prélèvement, soit les deux, sont pourvus d'un tel moyen de préhension. De préférence, l'on exerce une force de traction soit sur l'un, soit sur l'autre.

Afin d'exercer cette force de traction, et ainsi passer de ladite première position vers ladite deuxième position, il est nécessaire que le dispositif selon l'invention soit tenu, de préférence dans une zone située à l'opposé de celle présentant ledit moyen de préhension. L'opérateur peut tout simplement exercer ce maintien en tenant le dispositif selon l'invention dans sa/ses main(s). De préférence, l'opérateur exerce ladite force de traction d'une main et tient le dispositif selon l'invention de l'autre. Avantageusement, la zone du dispositif selon l'invention opposée à la zone comprenant au moins un moyen de préhension (« premier moyen de préhension ») possède également au moins un moyen de préhension (« deuxième moyen de préhension ») tel qu'une poignée, languette, tirette, etc.

Selon un mode de réalisation particulier, à la fois le premier et le deuxième moyens de préhension sont connectés au conteneur, de préférence tous deux connectés au moyen d'ouverture dudit conteneur. Ainsi, selon ce mode de réalisation particulier, une traction entre les premier et deuxième moyens de préhension induit ainsi l'ouverture du conteneur par déchirure, rupture, cassure, etc., de préférence au niveau du moyen d'ouverture.

Selon un mode de réalisation préféré, l'échantillon biologique est susceptible de contenir au moins un micro-organisme à analyser, ledit procédé comprenant, après l'étape a), l'étape suivante :
a') éventuellement mettre en contact ledit échantillon biologique avec au moins un moyen de culture adapté pour permettre la croissance du ou des micro-organisme(s) à analyser (ce moyen de culture, de préférence sous forme déshydratée, étant avantageusement disposé dans l'enceinte du contenant)
ledit procédé comprenant, après l'étape b) et avant l'étape c), l'étape suivante :
b') éventuellement placer ledit dispositif dans des conditions aptes à permettre la croissance du ou des micro-organisme(s) à analyser,
dans lequel, à l'étape c), l'on exerce une force, par exemple de type traction ou rotation, sur au moins une partie dudit moyen de prélèvement et/ou au moins une partie dudit moyen d'entrave d'analyse, afin de passer de ladite première position, dans laquelle l'analyse dudit moyen de prélèvement est malaisée ou impossible, de préférence impossible, vers ladite deuxième position dans laquelle l'analyse dudit moyen de prélèvement est possible, en vue d'effectuer l'analyse dudit moyen de prélèvement sans sortir ledit moyen de prélèvement dudit dispositif, de préférence ledit procédé comprenant l'étape a') et éventuellement l'étape b').

L'étape b') est uniquement nécessaire si la croissance du ou des micro-organisme(s) à analyser (micro-organisme(s) cible(s)) requiert des conditions particulières, notamment en termes de température, hygrométrie, luminosité, etc. ; la température étant par exemple un facteur déterminant concernant la croissance des micro-organismes. Toutefois, si l'opérateur met en oeuvre le dispositif et le procédé selon la présente invention sur un territoire possédant un climat clément (par exemple un climat tropical), il peut ne pas être nécessaire d'utiliser une étuve pour effectuer l'incubation de l'échantillon biologique comprenant un ou des micro-organisme(s) à analyser et dudit moyen de culture à une température donnée.

Par « moyen de culture adapté pour permettre la croissance du ou des micro-organisme(s) à analyser », l'on entend, au sens de la présente invention un moyen permettant de favoriser et/ou d'orienter la culture du ou des micro-organismes cibles/recherchés. Ce moyen de culture peut être, par exemple, un agent sélectif, tel qu'un ou plusieurs antibiotique(s), destiné(s) à améliorer la sélectivité de l'analyse (en éliminant tout ou partie des micro-organismes non désirés (flore annexe). L'emploi d'un ou plusieurs antibiotique(s), peut également être justifié par le fait que le ou les micro-organisme(s) cible(s), sont des micro-organismes antibio-résistants, à savoir résistants audit ou auxdits antibiotique(s).

Le moyen de culture peut également être un élément nutritif, par exemple sélectionné parmi les vitamines, peptones, hydrates de carbone, etc., destiné à favoriser la fonction première d'enrichissement en micro-organismes cibles de l'échantillon biologique testé.

Un tel moyen de culture peut comprendre ou consister en un milieu de culture, à savoir un milieu comprenant tous les éléments nécessaires à la survie et/ou à la croissance des micro-organismes et, en particulier des micro-organismes recherchés (par exemple de l'eau tamponnée peptonée). Le milieu de culture peut contenir d'éventuels additifs, par exemple : des peptones, un ou plusieurs facteurs de croissance, des hydrates de carbone, un ou plusieurs agents sélectifs, des tampons, un ou plusieurs gélifiants, une ou plusieurs vitamines, etc. Ce milieu de culture peut se présenter sous forme liquide ou gélifiée prête à l'emploi, à savoir prête à être ensemencée en tube, en flacon ou sur boîte de Pétri. L'expression « milieu de culture » englobe bien évidemment les milieux et bouillons d'enrichissement.

Selon un mode de réalisation particulièrement préféré, ledit moyen de culture est préalablement disposé dans l'enceinte du contenant, de préférence sous forme déshydratée. L'échantillon biologique est donc mis en contact avec le moyen de culture après introduction de l'échantillon biologique au sein dudit contenant.

La mise en contact d'un échantillon biologique avec au moins un moyen de culture tel que mentionné précédemment n'est pas nécessaire pour ce qui concerne les échantillons biologiques susceptibles/suspectés d'être naturellement riches en micro-organismes à analyser (micro-organisme(s) cible(s)) comme par exemple le lait de vache (dans le cadre d'application vétérinaire) ou de selles liquides telles que des diarrhées cholériques dans le cadre d'applications cliniques. Lorsque l'échantillon biologique à analyser est suffisamment riche en micro-organismes et, plus précisément, susceptible/suspecté d'être naturellement riches en micro-organisme(s) cible(s), l'utilisation d'un moyen de culture s'avère superflue et l'incubation peut être considérablement réduite dans sa durée.

En revanche, l'utilisation de moyen(s) de culture (et en particulier de milieu(x) de culture tel(s) que des bouillons de pré-enrichissement et/ou d'enrichissement) s'avère particulièrement importante en ce qui concerne les échantillons biologiques susceptibles/suspectés de contenir une quantité minime de micro-organisme(s) cible(s), par exemple de l'ordre de quelques cellules dans l'échantillon. L'utilisation de moyen(s) de culture et en particulier de milieu(x) de culture, a pour but de promouvoir la croissance des micro-organismes cibles dans les échantillons biologiques, si possible tout en limitant la croissance des flores non cibles.

Avantageusement, ledit moyen de prélèvement comprend au moins un moyen de capture de micro-organisme(s), tel qu'un support de capture apte à capturer le ou les micro-organisme(s) recherché(s), adapté pour être analysé à l'étape d), et/ou à l'étape e).

Ledit moyen de capture de micro-organisme(s) a été précédemment défini.

Selon un mode de réalisation particulièrement avantageux, à l'étape d), l'analyse dudit moyen de prélèvement est réalisée par lecture visuelle ou optique (par exemple via au moins un moyen de lecture optique tel qu'une caméra ou un spectroscope de Raman positionné à l'extérieur dudit contenant), de préférence par lecture visuelle.

Avantageusement, ledit échantillon biologique est mis en contact avec un système de révélation apte à permettre ou à favoriser ladite lecture visuelle ou optique par exemple lors de l'étape a).

Un tel système de révélation est bien connu de l'homme du métier et a été défini supra.

Selon un mode de réalisation particulièrement avantageux, le procédé selon l'invention comprend successivement les étapes d) et d'), dans lequel l'étape d'analyse e) est une étape de confirmation d'analyse visant à confirmer ou infirmer le résultat d'analyse obtenu à l'étape d).

L'analyse dudit moyen de prélèvement est effectuée, à l'étape d), dans ladite deuxième position (« position d'analyse »), cette deuxième position correspondant ou non à la position de désolidarisation. Dans le cas où cette deuxième position correspond à la position de désolidarisation, une fois l'analyse du moyen de prélèvement effectuée, l'on peut séparer ledit moyen de prélèvement par séparation hermétique (par exemple par thermoscellage et coupure), aux fins d'analyse ultérieure (analyse additionnelle et/ou confirmation).

A contrario, si, dans ladite position d'analyse (deuxième position), le moyen de prélèvement n'est pas positionné dans ladite position de désolidarisation, l'on exerce une force, par exemple de type traction ou rotation, sur ledit moyen de prélèvement, afin de venir le positionner dans sa position de désolidarisation. Une fois ceci effectué, l'on peut valablement effectuer l'étape d').

Un autre objet de l'invention concerne un dispositif adapté pour recevoir au moins un échantillon biologique et effectuer le transfert sécurisé (à savoir en limitant ou supprimant les risques de contamination de l'environnement) d'au moins une partie dudit échantillon biologique, ledit dispositif comprenant :
- au moins un contenant, de préférence étanche, adapté pour recevoir ledit échantillon biologique, ledit contenant comprenant au moins un moyen de remplissage tel qu'un orifice et au moins un moyen de fermeture permettant de fermer ledit moyen de remplissage, de préférence de manière hermétique,
- au moins un moyen de prélèvement mobile, connecté audit contenant, ledit moyen de prélèvement comprenant au moins une paroi, ledit moyen de prélèvement étant adapté pour passer d'une première position (position de protection), dans laquelle ladite au moins une paroi est protégée à l'intérieur dudit contenant, vers une deuxième position (position de désolidarisation), dans laquelle ladite au moins une paroi fait au moins partiellement, et de préférence totalement, saillie à l'extérieur dudit contenant,
ledit moyen de prélèvement étant en outre adapté pour :
- recevoir, dans ladite deuxième position (position de désolidarisation), au moins une partie dudit échantillon biologique dans ladite saillie dudit moyen de prélèvement,
- être désolidarisé dudit contenant, de préférence de manière hermétique, dans ladite deuxième position (position de désolidarisation), afin d'effectuer le transfert de ladite partie dudit échantillon biologique (vers un autre contenant).

L'intégrité du moyen de prélèvement, et plus particulièrement de sa ou ses paroi(s), est assurée dans la position de protection par le fait que ledit moyen de prélèvement est positionné à l'intérieur du contenant dans ladite position. Ainsi, le dispositif selon l'invention peut être stocké et/ou transporté sans risquer une dégradation du moyen de prélèvement. Ceci représente un avantage significatif, dans la mesure où cela garantit un bon fonctionnement du dispositif selon l'invention et, de manière encore plus avantageuse, évite les risques de contaminations de l'environnement (en limitant voire supprimant les risques de fuites au niveau de la ou des paroi(s) du moyen de prélèvement) lors de l'introduction d'au moins une partie de l'échantillon biologique dans ledit moyen de prélèvement.

Le moyen de prélèvement désolidarisé du contenant, après désolidarisation (de préférence hermétique, par exemple par thermoscellage et coupure), peut être envoyé à un laboratoire ou transporter vers ce dernier de manière sécurisée, par exemple aux fins d'analyse. De manière générale, la partie de l'échantillon contenue dans le moyen de prélèvement désolidarisé peut, aisément et sans risques de contamination, être transférée vers un autre contenant.

Selon un mode de réalisation préféré, ladite paroi consiste en une membrane flexible, adaptée pour être invaginée à l'intérieur du contenant dans ladite première position (position de protection) et au moins partiellement évaginée à l'extérieur du contenant dans ladite deuxième position (position de désolidarisation).

Cette membrane flexible consiste, au sens de la présente invention, en une membrane/paroi souple et hermétique afin d'éviter les risques de contamination microbiens.

La première position (position de protection) est une position dans laquelle ladite membrane flexible est invaginée à l'intérieur du contenant, à savoir a pénétré par retournement en doigt de gant à l'intérieur dudit contenant (forme en doigt de gant retourné).

Le passage de la position de protection à la position de désolidarisation s'effectue par retournement comme un doigt de gant de ladite membrane flexible, laquelle, en position évaginée (position de désolidarisation), fait saillie à la surface dudit contenant dans une forme de doigt de gant.

Tel qu'indiqué précédemment, cette forme en doigt de gant invaginé à l'intérieur du contenant permet de protéger l'intégrité de ladite membrane flexible dans ladite première position, qui représente la position dans laquelle le dispositif selon l'invention est stocké et/ou transporté. Cette forme en doigt de gant invaginé du moyen de prélèvement permet donc de limiter les risques de déchirure, rupture ou autres accrocs dans la membrane flexible constituant le moyen de prélèvement lors du stockage du dispositif selon l'invention et du transport de ce dernier.

Le passage dudit moyen de prélèvement de la position de protection vers la position de désolidarisation peut être effectué, par exemple, en appliquant une force de traction sur un moyen de préhension connecté à la surface externe de ladite membrane flexible ou, de manière préférée, en générant une surpression d'air (par exemple par compression d'au moins une paroi flexible dudit contenant) à l'intérieur dudit contenant, de façon à déclencher l'« évagination » dudit moyen de prélèvement, à savoir le retournement en doigt de gant de ladite membrane flexible. Bien évidemment, et en particulier dans le mode de réalisation selon lequel ladite au moins une paroi flexible est adaptée pour être comprimée afin d'induire l'évagination du moyen de prélèvement par surpression d'air, la ou les paroi(s) et membrane flexibles, ainsi que, plus généralement, les différents constituants du dispositif selon l'invention, sont soigneusement sélectionnés pour que leur intégrité soit préservée lors du passage de la position invaginée vers la position évaginée. Ceci est important afin de garantir la sécurité sanitaire de l'opérateur et de son environnement.

Dans cette position de désolidarisation, le moyen de prélèvement est adapté pour être désolidarisé dudit contenant, de préférence de manière hermétique (par exemple par thermoscellage et coupure), afin d'effectuer le transfert de tout ou partie (de préférence d'une partie aliquote) de l'échantillon biologique après que ce dernier a été introduit au sein du moyen de prélèvement.

L'aliquote de l'échantillon biologique d'intérêt peut être introduit dans le doigt de gant formé par le moyen de prélèvement évaginé par tout moyen, par exemple simplement en inclinant suffisamment le dispositif selon l'invention pour qu'une partie de l'échantillon biologique puisse couler dans le moyen de prélèvement évaginé. Une alternative afin d'introduire l'aliquote désiré au sein du moyen de prélèvement dans ladite position de désolidarisation consiste, par exemple, à comprimer le contenant avec une intensité suffisamment importante pour qu'une partie de l'échantillon biologique (et éventuellement du milieu de culture) puisse être transférée dudit contenant vers le moyen de prélèvement en position de désolidarisation (en forme de doigt de gant comme indiqué précédemment). Dans ce dernier cas, il est nécessaire qu'au moins une paroi dudit contenant soit déformable (par exemple soit réalisée au moins partiellement en matière souple ou flexible), de manière à ce que la compression appliquée au niveau de cette/ces paroi(s) déformable(s) puisse déformer cette dernière et ainsi induire le transfert d'au moins une partie de l'échantillon biologique (et éventuellement du milieu de culture) via une élévation du niveau de ce dernier au sein dudit contenant sans danger pour l'opérateur et l'environnement. Avantageusement, ladite membrane flexible comprend au moins un moyen de capture d'information biologique et/ou physico-chimique, ledit moyen de capture d'information biologique et/ou physico-chimique étant positionné sur la surface de ladite membrane flexible dans une position adaptée pour être en contact avec l'échantillon biologique dans ladite première position (position de protection) et n'être plus en contact avec ledit échantillon biologique dans ladite deuxième position (position de désolidarisation).

Le positionnement dudit moyen de capture d'information biologique et/ou physico-chimique sur la surface de ladite membrane flexible est défini pour que ce moyen de capture soit en contact avec l'échantillon biologique lorsque ledit moyen de prélèvement est invaginé dans le contenant (dans la position de protection), à savoir présente une forme de doigt de gant retourné, et pour n'être plus en contact avec ledit échantillon biologique en position au moins partiellement évaginée à l'extérieur du contenant (dans la position de désolidarisation), à savoir en forme de doigt de gant. Si l'on poursuit l'analogie avec une forme en doigt de gant retourné/doigt de gant, le moyen de capture d'information biologique et/ou physico-chimique est de préférence positionné au niveau de l'extrémité du doigt de gant retourné en contact avec l'échantillon biologique en première position (invaginée). Ce mode de réalisation est particulièrement préféré puisqu'il offre la possibilité, outre le fait de pouvoir éventuellement prélever un aliquote de l'échantillon biologique d'intérêt aux fins de transfert comme décrit précédemment, d'effectuer la capture d'au moins une information biologique et/ou physico-chimique d'intérêt en première position (position invaginée), en laissant ledit moyen de capture en contact avec l'échantillon biologique d'intérêt durant un laps de temps suffisant pour effectuer la capture de la ou des informations biologique(s) et/ou physico-chimique(s) d'intérêt, puis, dans un second temps (lorsque l'opérateur estime que le moyen de capture est resté en contact avec l'échantillon biologique durant un laps de temps suffisant), d'effectuer le transfert dudit moyen de capture en exerçant une force, par exemple de type traction ou rotation, sur ledit moyen de prélèvement permettant d'obtenir son déplacement de sa première position (invaginée) vers sa position de désolidarisation (évaginée). Une fois la position de désolidarisation atteinte, l'opérateur peut, s'il le désire, séparer (désolidariser) hermétiquement le moyen de prélèvement contenant ledit moyen de capture d'information biologique et/ou physico-chimique (ainsi qu'éventuellement un aliquote d'échantillon biologique) afin d'envoyer ledit moyen de prélèvement séparé du contenant à un laboratoire, en vue, par exemple, d'effectuer l'analyse et/ou la confirmation d'analyse dudit moyen de capture « emprisonné » à l'intérieur du moyen de prélèvement scellé par thermoscellage. Tel qu'indiqué précédemment, et si cela est conforme au souhait de l'opérateur, outre le moyen de capture d'information biologique et/ou physico-chimique, le moyen de prélèvement hermétiquement séparé dudit contenant peut comprendre un aliquote issu de l'échantillon biologique initialement introduit au sein du contenant. Ceci peut notamment s'avérer intéressant si l'on souhaite (re)mettre en culture tout ou partie de la matière biologique, en particulier la matière biologique, se trouvant dans cet aliquote.

Selon un mode de réalisation particulier, avant séparation hermétique du moyen de prélèvement, ce dernier peut être analysé dans ladite position de désolidarisation. Dans cette configuration, le moyen de capture d'information biologique et/ou physico-chimique est, de préférence, un moyen de capture de micro-organisme(s) tel que défini précédemment, pouvant avantageusement être analysé par lecture visuelle ou optique. Dans ladite position de désolidarisation, la membrane flexible constituant le moyen de prélèvement étant, dans ce mode de réalisation, translucide ou transparente, de préférence transparente, afin de permettre la lecture visuelle ou optique du moyen de capture de micro-organisme(s).

Ainsi, le dispositif selon la présente invention offre une latitude d'utilisation importante à l'opérateur. En effet, ce dernier peut, s'il le désire :
- utiliser simplement ledit dispositif pour transférer un aliquote de l'échantillon biologique d'intérêt vers un autre contenant afin, par exemple, d'effectuer des analyses plus approfondies ;
- transférer au moins un moyen de capture d'information biologique et/ou physico-chimique ayant été préalablement mis en contact avec l'échantillon biologique à analyser vers un autre contenant afin d'effectuer une analyse et/ou confirmation d'analyse additionnelle(s) ;
- effectuer l'analyse, de préférence par lecture visuelle ou optique, dudit moyen de capture d'information biologique et/ou physico-chimique présent sur ledit moyen de prélèvement, dans la position de désolidarisation, éventuellement en préalable à un tel transfert, etc.

La forme préférentielle du moyen de prélèvement selon l'invention dans sa première position (position invaginée) à savoir une forme en doigt de gant retourné dont au moins l'extrémité est en contact avec l'échantillon biologique d'intérêt dans ladite première position est particulièrement avantageuse, lorsqu'au moins un moyen de capture d'information biologique et/ou physico-chimique est positionné à cette extrémité pour être en contact avec l'échantillon biologique dans la position du départ puisque l'opérateur peut glisser son doigt à l'intérieur du moyen de prélèvement en forme de doigt de gant retourné afin de déplacer le moyen de capture d'information biologique et/ou physico-chimique vers une ou plusieurs zones d'intérêt. Ceci peut présenter un avantage notamment si l'échantillon biologique est sous forme semi-solide (tel qu'un échantillon de selles) ou si l'échantillon n'est pas correctement/suffisamment homogénéisé. Ainsi, l'opérateur peut manuellement diriger le moyen de capture d'information biologique et/ou physico-chimique vers/dans les zones de l'échantillon biologique ou l'on peut légitimement s'attendre à ce que l'information biologique et/ou physico-chimique recherchée soit présente en quantité plus importante.

Le moyen de capture d'information biologique et/ou physico-chimique est tel que défini précédemment. De préférence, ledit moyen de capture d'information biologique et/ou physico-chimique comprend un moyen de capture de micro-organisme(s) tel qu'un support de capture apte à capturer le ou les micro-organisme(s) recherché(s), également défini supra.

Selon un mode de réalisation particulier, ledit moyen de capture est adapté pour être analysé, de préférence dans ladite position de désolidarisation, par lecture visuelle ou optique, de préférence par lecture optique via au moins un moyen de lecture optique positionné à l'extérieur du contenant tel qu'une caméra ou un spectroscope de Raman.

L'invention concerne également un procédé permettant d'effectuer le transfert sécurisé d'au moins une partie d'un échantillon biologique, ledit procédé mettant en oeuvre le dispositif selon l'invention, ledit procédé comprenant les étapes suivantes :
a) introduire, par ledit moyen de remplissage, au moins un échantillon biologique au sein dudit contenant,
b) fermer ledit moyen de remplissage, de préférence de manière hermétique, à l'aide dudit moyen de fermeture,
c) exercer une force, par exemple de type traction ou compression, sur ledit moyen de prélèvement afin de passer de ladite première position (position de protection) vers ladite deuxième position (position de désolidarisation),
d) recevoir, dans ladite deuxième position (position de désolidarisation), au moins une partie dudit échantillon biologique dans ladite saillie dudit moyen de prélèvement,
e) désolidariser, dans ladite deuxième position (position de désolidarisation), ledit moyen de prélèvement, de préférence par séparation hermétique, par exemple par thermoscellage et coupure, en vue d'effectuer le transfert de ladite partie dudit échantillon biologique.

De préférence, ledit procédé comprend entre les étapes d) et e), ou après l'étape e), l'étape suivante:
d') analyser ladite partie dudit échantillon biologique, de préférence par lecture visuelle ou optique, avantageusement par lecture optique.

Selon un mode de réalisation particulier, à l'étape c), l'on exerce une force de traction sur au moins un moyen de préhension connecté audit moyen de prélèvement.

Selon un autre mode de réalisation particulier, à l'étape c), l'on exerce une force de compression sur ledit moyen de prélèvement, par exemple en créant une surpression d'air à l'intérieur dudit contenant.

Selon un mode de réalisation préféré, ce procédé comprend, lors de l'étape a) ou postérieurement à cette dernière, l'étape suivante :
a') mettre en contact ledit échantillon biologique avec au moins un moyen de culture adapté pour permettre la croissance d'un ou plusieurs micro-organisme(s) cibles,
ledit procédé comprenant, après l'étape b) et avant l'étape c), l'étape suivante :
b') éventuellement placer ledit dispositif dans des conditions aptes à permettre la croissance du ou des micro-organisme(s) cibles.

Selon un mode de réalisation particulier, ledit moyen de prélèvement comprend au moins un moyen de capture de micro-organisme(s), tel qu'un support de capture apte à capturer le ou les micro-organisme(s) recherché(s).

Le moyen de culture est tel que défini précédemment.

### Brève description des dessins

L'invention, sa fonctionnalité, ses applications ainsi que ses avantages seront mieux appréhendés à la lecture de la présente description, faite en référence aux figures, dans lesquelles :
- la figure 1 montre une vue en perspective, et partiellement en coupe, d'un premier mode de réalisation du dispositif selon la présente invention,
- la figure 2 représente le dispositif selon la figure 1, en vue de coupe, dans une première position,
- la figure 3 montre le dispositif selon la figure 1, en vue de coupe, dans une deuxième position,
- la figure 4 représente le dispositif selon la figure 3, après un procédé de thermosoudage (thermoscellage) permettant de préparer la désolidarisation du moyen de prélèvement d'information biologique et/ou physico-chimique,
- les figures 4a et 4b montrent le dispositif selon la figure 4, après la désolidarisation dudit moyen de prélèvement,
- la figure 5 représente un détail du dispositif selon les figures 1 - 4, comprenant un moyen d'inviolabilité,
- la figure 6 est une vue en coupe montrant un deuxième mode de réalisation du dispositif selon l'invention,
- la figure 7 représente un troisième mode de réalisation du dispositif selon l'invention, dans une première position,
- la figure 8 montre le dispositif selon la figure 7, dans une deuxième position,
- la figure 9 représente un quatrième mode de réalisation du dispositif selon l'invention, dans une première position,
- la figure 10 montre le dispositif selon la figure 9, dans une deuxième position,
- la figure 11 est une vue en perspective représentant le moyen de prélèvement ainsi que le cache du dispositif selon la figure 9.

### Description détaillée de l'invention

La description détaillée ci-après a pour but d'exposer l'invention de manière suffisamment claire et complète, notamment à l'aide de références à des figures, mais ne doit en aucun cas être regardée comme limitant l'étendue de la protection aux modes de réalisation particuliers objet desdites figures.

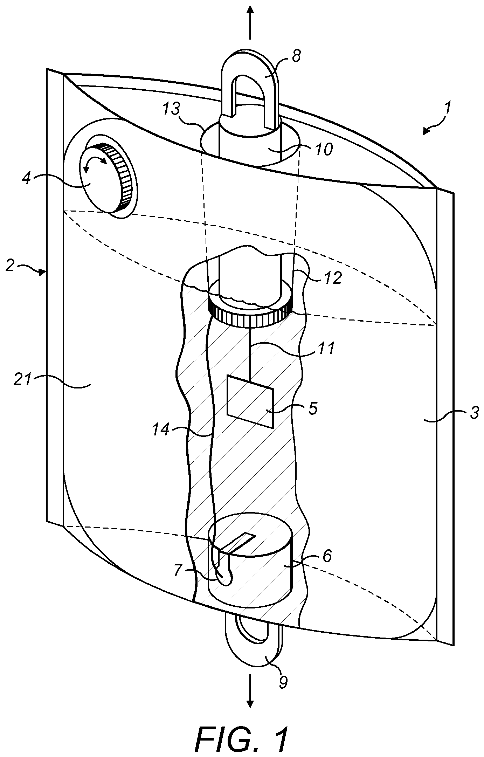

La figure 1 montre un premier mode de réalisation du dispositif 1 selon la présente invention. Le dispositif 1 comprend un contenant 2 se présentant sous la forme d'un sac adapté pour incuber un échantillon biologique. Le contenant 2 comprend, par exemple, du papier rigide, du carton, du polyéthylène, du polychlorure de vinyle, du polypropylène, du polystyrène, du polyéthylène téréphtalate, ainsi que toute combinaison adaptée de ces matériaux et/ou tout autre matériau (de préférence matériaux biosourcés) afin que le contenant 2 possède des propriétés physiques suffisantes notamment en termes de tenue et d'étanchéité suffisante du contenant 2. Afin de procéder à l'incubation dudit échantillon, l'on introduit, au sein du contenant 2, un milieu de culture tel qu'un bouillon d'enrichissement 3. Le contenant 2 est pourvu d'une ouverture pouvant passer d'une position « ouverte » à une position « fermée » grâce à un bouchon 4. Lorsque le bouchon 4 est ôté, l'ouverture peut permettre l'introduction d'un échantillon biologique à l'intérieur du contenant 2. Après l'introduction dudit échantillon biologique, le bouchon 4 peut servir à obturer l'ouverture, de préférence de façon totalement hermétique. Lorsque l'échantillon biologique a été introduit à l'intérieur du contenant 2, le milieu de culture, tel que le bouillon d'enrichissement 3, est utilisé pour incuber ledit échantillon biologique à l'intérieur du contenant 2.

Le dispositif 1 est pourvu d'un moyen de prélèvement 5. Selon le mode de réalisation représenté sur la figure 1, le moyen de prélèvement 5 comprend un support de capture fonctionnalisé par un partenaire de liaison spécifique du micro-organisme à détecter (micro-organisme cible), tel qu'une bactérie. Le moyen de prélèvement 5 est adapté pour une analyse de type lecture visuelle ou optique du résultat de l'analyse microbiologique effectuée à l'aide du dispositif 1 selon l'invention.

Le dispositif 1 est pourvu d'un compartiment 6 comprenant, en son intérieur, une certaine quantité d'agent désinfectant, présent de préférence sous forme concentrée. Le compartiment 6 est pourvu d'un moyen de rupture 7 adapté pour permettre l'ouverture dudit compartiment 6 et autoriser ainsi la propagation du contenu de ce compartiment 6 à l'intérieur du contenant 2. Le compartiment 6 et le moyen de rupture 7 sont adaptés pour permettre au dispositif 1 selon la figure 1 de réaliser une étape de désinfection du contenu dudit dispositif 1 de manière quasi-concomitante ou concomitante à la lecture visuelle ou optique du moyen de prélèvement 5. En d'autres termes, cela signifie que lorsqu'un opérateur du dispositif 1 manipule ledit dispositif vers une position permettant la lecture visuelle ou optique du moyen de prélèvement 5, le processus de désinfection s'effectue de façon quasi-simultanée ou simultanée.

Le fonctionnement du dispositif 1 selon la figure 1 est assuré grâce au fait que les parois 21 du contenant 2 sont opaques. En effet, dans la première position telle que montrée en figure 1, la lecture visuelle ou optique du moyen de prélèvement 5 est difficile voire impossible, de préférence impossible. Afin soit de faciliter la lecture du moyen de prélèvement 5, soit de la rendre possible (de préférence la rendre possible), l'opérateur désireux d'effectuer cette lecture visuelle ou optique doit nécessairement manipuler le moyen de prélèvement 5 afin de le déplacer vers une deuxième position, dans laquelle la lecture visuelle ou optique dudit moyen de prélèvement 5 pourra être effectuée. Ces parois opaques 21 constituent donc un moyen d'entrave d'analyse au sens de la présente invention.

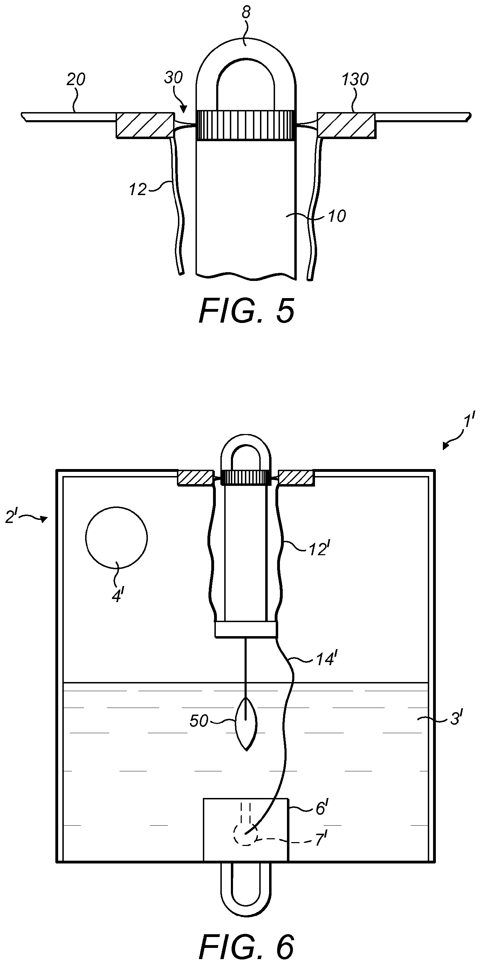

La manipulation du moyen de prélèvement 5 selon la figure 1 est possible grâce à une première et une deuxième poignées 8, 9 présentes sur le dispositif 1. La première poignée 8 est connectée, via une languette 10, et une tige 11, au moyen de prélèvement 5. La partie inférieure de la languette 10 est connectée, via une paroi/membrane flexible 12, au contour 13 d'une ouverture dans la paroi supérieure du contenant 2. A noter que le contour 13 peut également comporter un élément renforcé tel qu'un anneau (cf. figure 5 infra).

La deuxième poignée 9 est connectée à l'extérieur du contenant 2 et aux parois inférieures dudit contenant 2. Lorsqu'un opérateur tire sur les poignées 8 et 9, dans la direction indiquée par des flèches sur la figure 1, le moyen de prélèvement 5 peut être déplacé en direction du contour 13 et libéré, de l'intérieur du contenant 2 vers l'extérieur du contenant 2.

Comme indiqué sur la figure 1, la partie inférieure de la languette 10 est connectée, grâce à un fil 14, à l'élément de rupture 7 du compartiment 6 (conteneur). Ainsi, l'on effectue le déplacement du moyen de prélèvement 5 en direction du contour 13 via la languette 10, ladite connexion de la languette 10 au fil 14 a pour effet d'induire, dans un même temps, l'ouverture du compartiment 6 en déplaçant l'élément de rupture 7 dudit compartiment 6. Cela signifie que dès que l'opérateur effectue un déplacement du moyen de prélèvement 5 pour rendre sa lecture visuelle ou optique possible, la désinfection quasi-concomitante ou concomitante de tout ou partie (de préférence de la totalité) de l'intérieur du contenant 2 est assurée par la libération et la propagation de l'agent désinfectant présent à l'intérieur du compartiment 6.

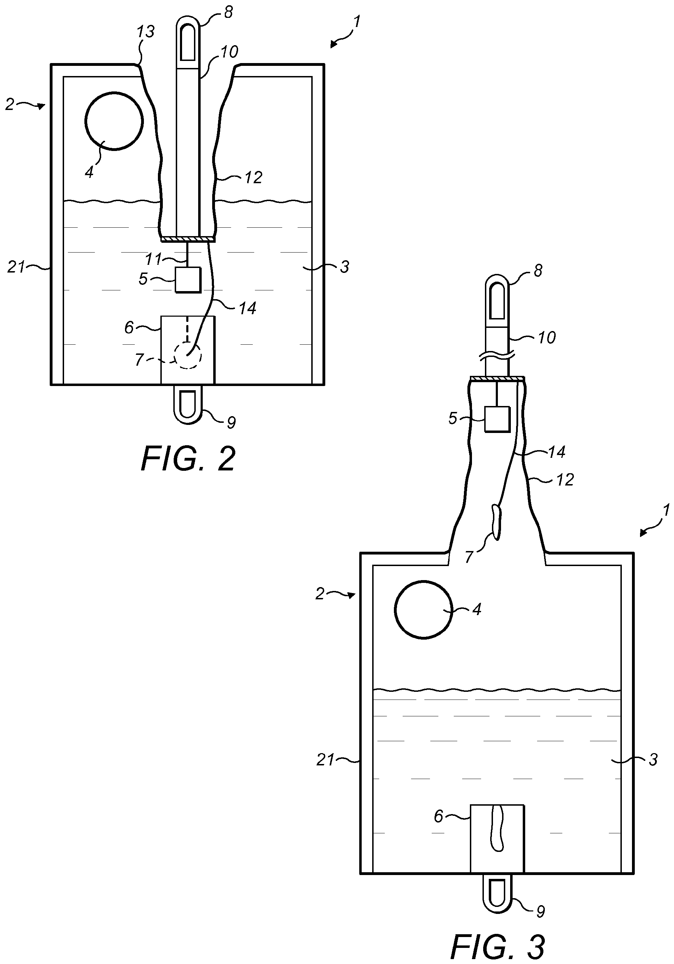

Les figures 2 et 3 montrent le fonctionnement du dispositif 1 selon la figure 1 et les deux positions dudit dispositif 1 (à savoir, respectivement les première et deuxième positions).

La figure 2 représente le dispositif 1 selon l'invention en vue de coupe. Le dispositif 1 selon la figure 2 est montré dans sa première configuration, identique à la configuration représentée dans la figure 1.

La figure 2 indique clairement que le moyen de prélèvement 5 est, dans la première configuration du dispositif 1, en contact avec le milieu de culture tel qu'un bouillon d'enrichissement 3. Cela signifie qu'à partir du moment où l'échantillon biologique est introduit à l'intérieur du contenant 2, le moyen de prélèvement 5 permet la capture d'une éventuelle bactérie cible. La bactérie cible capturée au niveau du support de capture fonctionnalisé pourra être analysée dans la deuxième position par lecture visuelle ou optique.

La figure 2 montre également clairement la présence d'une paroi/membrane souple 12 entre la partie inférieure de la languette 10 et le contour 13 d'une ouverture dans la paroi supérieure du contenant 2. Par ailleurs, la figure 2 représente la connexion entre la partie inférieure de la languette 10 et le moyen de rupture 7 présent dans le compartiment 6 qui s'effectue à l'aide d'un fil 14.

Le dispositif 1 peut être manipulé et déplacé de sa première position (représentée sur la figure 2), dans laquelle ladite paroi/membrane souple 12 présente une forme de doigt de gant retourné à l'intérieur du contenant 2 (position invaginée), vers sa deuxième position (représenté sur la figure 3), dans laquelle ladite paroi/membrane souple 12 présente une forme de doigt de gant faisant saillie à la surface du contenant 2 (position évaginée).

Sur la figure 3, l'on observe que le moyen de prélèvement 5 a été déplacé de l'intérieur du contenant 2 vers son extérieur. Le moyen de prélèvement 5 reste enveloppé par une paroi/membrane souple 12 en position «évaginée ». Selon l'invention, la paroi/membrane 12 est réalisée à l'aide d'un matériau transparent. Cela signifie que, dans la deuxième position représentée sur la figure 3, la lecture visuelle ou optique du moyen de prélèvement 5 est possible.

Tel que représenté sur la figure 3, après passage de la première position (cf. figure 2) à la deuxième position (cf. figure 3), la partie inférieure de la languette 10 est, toujours connectée à l'élément de rupture 7 grâce au fil 14. En déplaçant la languette 10, l'élément de rupture 7 a été déplacé, ledit déplacement ayant eu comme effet l'ouverture du compartiment 6 à l'intérieur du contenant 2. L'ouverture du compartiment 6 permet la libération et la propagation de son contenu, c'est-à-dire de l'agent désinfectant présent à l'intérieur du compartiment 6, dans le milieu de culture tel que le bouillon d'enrichissement 3 présent à l'intérieur du contenant 2.

La connexion de la partie inférieure de la languette 10 à l'élément de rupture 7 permet de s'assurer que le contenu du contenant 2 est désinfecté et ce, de façon concomitante ou quasi-concomitante, dès que le moyen de prélèvement 5 est disponible pour une lecture visuelle ou optique. La lecture du moyen de prélèvement 5 contraint ainsi l'opérateur à désinfecter le contenu du dispositif 1 sans que l'opérateur n'ait à s'en soucier. Aucune action spécifique n'est requise de la part de l'opérateur en termes de désinfection du contenant 2. Après l'utilisation du dispositif 1 selon l'invention, le contenu est désinfecté et ne représente plus aucun danger biologique pour le stockage et/ou le transport avant sa destruction finale.

Il convient de noter que le moyen de prélèvement 5 peut comporter tout moyen de prélèvement adapté permettant de mesurer un ou plusieurs paramètres biologiques et/ou physico-chimiques d'un échantillon. Cela signifie que le moyen de prélèvement 5 peut comporter, outre un support de capture fonctionnalisé adapté pour capturer une bactérie cible, un élément adapté pour transférer une certaine quantité d'un échantillon biologique en vue d'une analyse ultérieure. Un tel élément peut comporter, par exemple, un élément spongieux (tel que du coton) ou compressible (tel qu'une éponge) positionné à l'extrémité d'une tige, adapté pour absorber une certaine quantité d'échantillon biologique. Un élément de ce type est représenté sur la figure 6.

Selon un mode de réalisation préféré de l'invention, le moyen de prélèvement 5 peut être désolidarisé/séparé du contenant 2 du dispositif selon l'invention en conservant le côté hermétique de l'enveloppe dudit moyen de prélèvement et en s'assurant que le contenant 2 reste hermétiquement fermé.

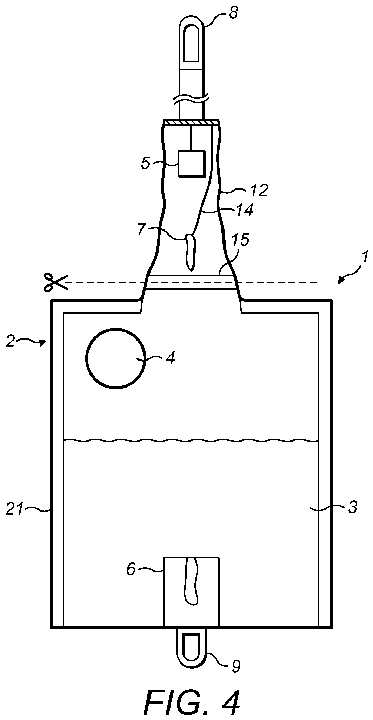

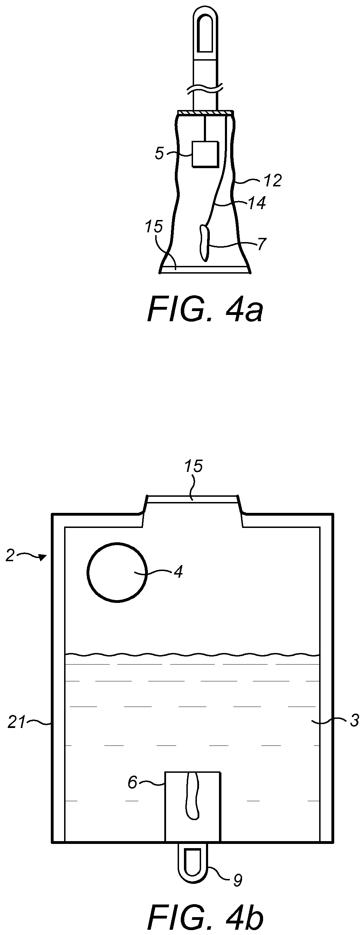

Les figures 4, 4a et 4b montrent une possibilité de séparer, de manière hermétique (étanche) une partie du dispositif 1 comprenant le moyen de prélèvement 5.

La figure 4 représente le dispositif 1 selon l'invention dans sa deuxième position. Le moyen de prélèvement 5 est enveloppé par la paroi/membrane souple 12. En vue de préparer la séparation du moyen de prélèvement 5 du contenant 2, éventuellement après que le moyen de prélèvement 5 a été analysé par lecture visuelle ou optique dans ladite deuxième position, une première étape consiste en un procédé de thermosoudage (thermoscellage) permettant de créer une zone de soudage 15. Cette zone de soudage 15 a pour but, dans un premier temps de fermer, de façon hermétique (à savoir d'obturer), la paroi supérieure du contenant 2. De façon simultanée, la paroi/membrane souple 12 est hermétiquement fermée en sa partie inférieure. Lorsque la zone de soudage 15 est créée, elle peut servir à séparer la partie comprenant le moyen de prélèvement 5 du contenant 2. Cette séparation est représentée sur les figures 4a et 4b. Dans un premier temps, le contenant 2, comprenant en son intérieur une quantité de matière biologique désinfectée, est hermétiquement scellé. La zone de soudage 15 garantit une fermeture hermétique de la partie comprenant le moyen de prélèvement 5, ce dernier étant lui-même hermétiquement fermé. Pour permettre ce procédé, le matériau utilisé pour réaliser la paroi 12 devrait être choisi pour sa capacité d'être utilisé dans un procédé de thermosoudage.

Le cas échéant, un opérateur peut procéder à l'introduction, à l'intérieur de la partie comprenant le moyen de prélèvement 5, d'une certaine quantité de matière biologique, en pratique d'une certaine quantité du milieu composé de l'échantillon biologique et du milieu de culture. La présence de la zone de soudage 15 garantit l'étanchéité de toute matière biologique enveloppée par la paroi 12 et la zone de soudage 15 et ce pendant le stockage et/ou le transport.

La figure 5 montre une partie du dispositif 1 selon l'invention dans un mode de réalisation comportant des moyens d'inviolabilité. Selon le mode de réalisation représenté sur la figure 5, le contour 13 comporte un anneau 130. Cet anneau 130 est réalisé, par exemple, en un matériau permettant la fixation, sur ledit anneau 130, d'une partie de la paroi supérieure 20 du contenant 2 et, d'autre part, de la paroi/membrane souple 12. Des moyens d'inviolabilité 30 sont présents entre l'anneau 130 et la partie supérieure de la languette 10. Ces moyens d'inviolabilité 30 consistent, par exemple, en des ponts en matière plastique permettant de connecter l'anneau 130 aux parties supérieures de la languette 10. Un premier objectif de ces moyens d'inviolabilité 30 consiste à permettre de positionner correctement la languette 10 à l'intérieur du contenant 2 avant son utilisation. Lorsque l'opérateur souhaite utiliser le dispositif 1 selon l'invention, les moyens d'inviolabilité sont détruits lors de la première utilisation, c'est-à-dire que leur présence ne gêne en aucun cas l'utilisation normale du dispositif 1 selon l'invention. En revanche, lorsque les moyens d'inviolabilité sont détruits, la preuve visible de l'utilisation du dispositif 1 selon l'invention est avérée. Tout opérateur est donc en mesure de contrôler, à l'oeil nu, si un dispositif 1 est vierge ou s'il a déjà été utilisé.

La figure 6 décrit un deuxième mode de réalisation du dispositif 1 selon l'invention. Le dispositif 1' selon la figure 6 comprend un moyen de prélèvement 50 ayant la forme d'une tige comprenant un moyen spongieux tel que du coton, ledit moyen spongieux présentant la capacité d'absorber au moins une certaine quantité de matière biologique diluée dans un milieu de culture, tel que le bouillon d'enrichissement 3' présent à l'intérieur du contenant 2'.

A l'inverse du moyen de prélèvement 5, tel que montré dans la figure 1, le moyen de prélèvement 50 n'est pas adapté pour capturer spécifiquement les micro-organismes cibles et permettre leur détection, par exemple, par analyse visuelle ou optique. En revanche, ce moyen de prélèvement 50 est adapté pour absorber une certaine quantité de matière biologique et transférer celle-ci à l'intérieur de la paroi 12' lorsque le dispositif 1' est déplacé de sa première position, telle que montrée dans la figure 6, vers sa deuxième position (position de désolidarisation/transfert, non représentée), de façon similaire aux manipulations telles que représentées sur les figures 2 et 3 et décrites en référence auxdites figures.

Comme décrit en faisant référence aux figures 4, 4a et 4b, l'enveloppe formée de la paroi/membrane souple 12 et la zone de soudage 15 peut être utilisée pour envelopper, de façon hermétique, le moyen de prélèvement 50, pour le transport ou encore pour effectuer une analyse ultérieurement.

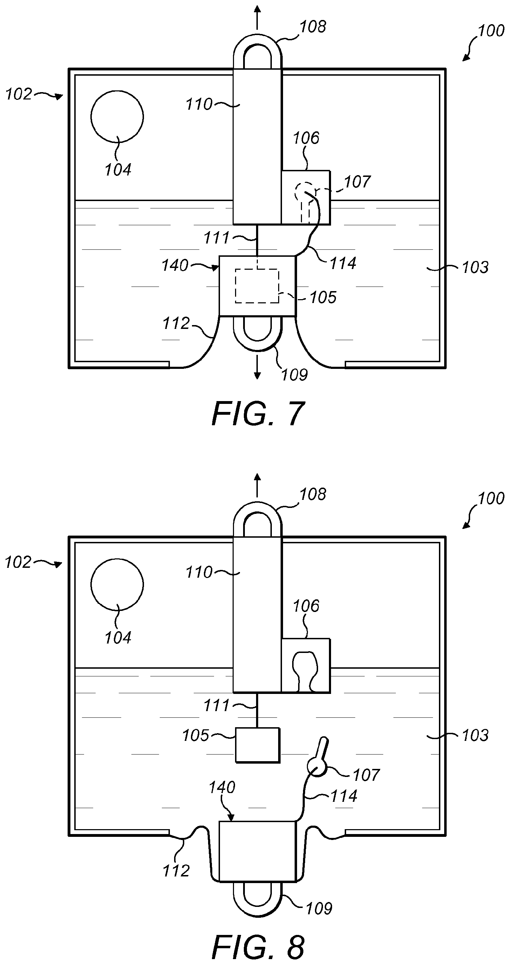

La figure 7 représente un troisième mode de réalisation du dispositif 100 selon l'invention. Le dispositif 100 comporte un contenant 102 adapté pour recevoir, en son sein, un milieu de culture tel qu'un bouillon d'enrichissement 103. De plus, un moyen de prélèvement 105 est présent à l'intérieur du contenant 102. Le dispositif 100 comporte une ouverture hermétiquement scellée grâce à un bouchon 104. Lorsque le bouchon 104 est ôté, un échantillon biologique peut être introduit à l'intérieur du contenant 102. Le moyen de prélèvement 105 est adapté pour détecter et capturer une bactérie cible à l'intérieur du contenant 102. Le moyen de prélèvement 105 est également adapté pour une analyse visuelle ou optique. Le dispositif 100 selon la figure 7 comprend un contenant 102 muni de parois partiellement transparentes. Cela signifie que l'analyse, visuelle ou optique, du moyen de prélèvement 105 est possible à travers les parois du contenant 102.

Afin d'imposer à un opérateur une utilisation spécifique du dispositif 100 selon la figure 7, le dispositif 100 comporte un cache 140 (représentant un moyen d'entrave d'analyse au sens de la présente invention), ledit cache 140 étant adapté pour former une paroi opaque qui rend la lecture visuelle ou optique du moyen de prélèvement 105 difficile, voire impossible (de préférence impossible). Le cache 140, présent à l'intérieur du dispositif 100, est amovible. Il peut être déplacé d'une première position, telle que montrée dans la figure 7, vers une deuxième position, telle que montrée dans la figure 8. Afin de réaliser une désinfection du contenu du contenant 102, de façon concomitante ou quasi-concomitante à la lecture optique ou visuelle du moyen de prélèvement 105, le cache 140 est fixé grâce à un fil 114 à un élément de rupture 107 présent sur un conteneur 106. Le conteneur 106 est adapté afin de recevoir, en son intérieur, une quantité d'agent désinfectant, de préférence sous forme concentrée. Le déplacement du cache 140, de sa position telle que montrée en figure 7 (« première position »), vers sa position telle que montrée en figure 8 (« deuxième position ») a pour effet de rendre visible le moyen de prélèvement 105, permettant ainsi la lecture visuelle ou optique dudit moyen de prélèvement 105. La connexion entre le cache 140 et l'élément de rupture 107, à l'aide du fil 114, a pour effet d'ouvrir le conteneur 106 et libérer son contenu à l'intérieur du contenant 102 dès lors que le cache 140 est déplacé de sa première position vers sa deuxième position. L'ouverture du conteneur 106 et la libération de son contenu sont montrées sur la figure 8.

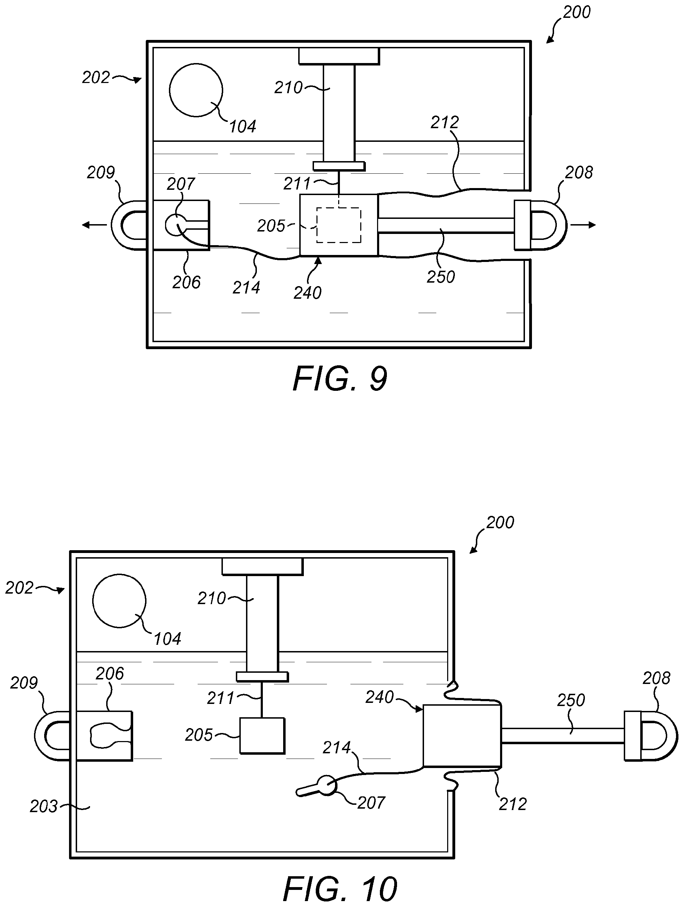

La figure 9 représente un quatrième mode de réalisation du dispositif selon l'invention, selon lequel un dispositif 200 selon l'invention comprend un contenant 202 adapté pour recevoir, en son intérieur, un milieu de culture tel qu'un bouillon d'enrichissement 203. Un moyen de prélèvement 205 est présent à l'intérieur du contenant 202. Ce moyen de prélèvement 205 est maintenu en place grâce à une languette 210 et à une tige 211. Le contenant 202 comprend des parois au moins partiellement transparentes permettant la visibilité du moyen de prélèvement 205 à travers les parois du contenant 202. En revanche, en vue d'imposer à un opérateur une étape de désinfection automatique, le dispositif 200 comprend un cache 240, amovible et pouvant être déplacé à l'aide d'une poignée 208 connectée audit cache à l'aide d'une tige 250. Le cache 240 peut être déplacé de sa position, telle que montrée dans la figure 9 (« première position »), vers sa position telle que montrée dans la figure 10 (« deuxième position ») en tirant sur les deux poignées 208 et 209 dans la direction indiquée à l'aide des flèches représentées sur la figure 9.

Le cache 240 est adapté pour rendre difficile, voire impossible (de préférence impossible) la lecture du moyen de prélèvement 205, dans la position telle que montrée dans la figure 9 (« première position »). La lecture du moyen de prélèvement 205 est possible dans la position telle que montrée dans la figure 10 (« deuxième position »). Le cache 240 est connecté, à l'aide d'un fil 214, à un élément de rupture 207. L'élément de rupture 207 est lui-même connecté à un conteneur 206 comportant une quantité d'agent(s) désinfectant(s), ledit désinfectant étant de préférence sous forme concentrée. Le déplacement de la tige 240, de sa position selon la figure 9 vers sa position selon la figure 10 a pour effet le déplacement de l'élément de rupture 207 afin de permettre au contenu du conteneur 206 de se propager à l'intérieur du contenant 202. L'ouverture du conteneur 206 est montrée dans la figure 10.

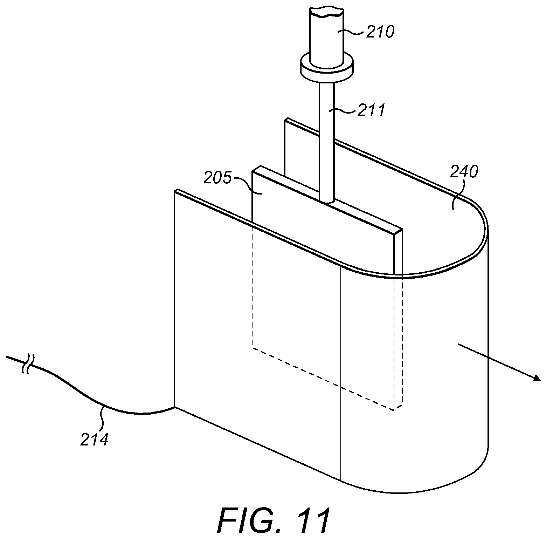

La figure 11 décrit un mode de réalisation particulier du cache 240, jouant le rôle de moyen d'entrave d'analyse au sens de la présente invention. Le cache 240 selon la figure 11 présente une forme courbée permettant de couvrir deux côtés du moyen de prélèvement 205.

Le dispositif et le procédé selon la présente invention permettent également d'effectuer des contrôles de stérilité notamment au sein des prélèvements alimentaires et environnementaux. Pour ce faire, l'on utilise, un dispositif selon la présente invention comprenant au moins un moyen de prélèvement, ledit moyen de prélèvement comportant des moyens de détection génériques de micro-organismes tels que des supports de capture fonctionnalisés avec des partenaires de liaison génériques de type anti-Gram-, anti-Gram+, etc.

## Revendications

1. Dispositif (1, 1', 100, 200) adapté pour recevoir au moins un échantillon biologique, ledit dispositif (1, 1', 100, 200) comprenant :
- au moins un contenant (2, 2', 102, 202), de préférence étanche, adapté pour recevoir ledit échantillon biologique, ledit contenant (2, 2', 102, 202) comprenant au moins un moyen de remplissage tel qu'un orifice et au moins un moyen de fermeture (4, 4', 104) permettant de fermer ledit moyen de remplissage, de préférence de manière hermétique,
- au moins un moyen de prélèvement (5, 50, 105, 205) positionné dans l'enceinte dudit contenant (2, 2', 102, 202), fixe ou mobile, ledit moyen de prélèvement (5, 50, 105, 205) étant adapté pour prélever de l'information biologique et/ou physico-chimique à partir dudit échantillon biologique, ledit moyen de prélèvement (5, 50, 105, 205) étant adapté pour être analysé après prélèvement de ladite information biologique et/ou physico-chimique et/ou placé dans une position de désolidarisation, et
- au moins un conteneur (6, 6', 106, 206), tel qu'une pochette ou un compartiment, comprenant au moins un agent désinfectant, ledit agent désinfectant étant relargable à l'intérieur du contenant (2, 2', 102, 202) après ouverture dudit conteneur (6, 6', 106, 206),
- lorsque ledit moyen de prélèvement (5, 50, 105, 205) est adapté pour être analysé après prélèvement de ladite information biologique et/ou physico-chimique, au moins un moyen d'entrave d'analyse (21, 140, 240) distinct dudit moyen de prélèvement (5, 50, 105, 205), fixe (21) ou mobile (140, 240), entravant partiellement ou totalement, de préférence totalement, l'analyse dudit moyen de prélèvement (5, 50, 105, 205),
dans lequel au moins une partie dudit moyen de prélèvement (5, 50, 105, 205) et/ou au moins une partie du moyen d'entrave d'analyse (140, 240) est/sont adaptée(s) pour passer d'une première position, dans laquelle l'analyse dudit moyen de prélèvement (5, 50, 105, 205) et/ou la désolidarisation dudit moyen de prélèvement (5, 50, 105, 205) dudit contenant (2, 2', 102, 202) est/sont malaisé(e)(s) ou impossible(s), de préférence impossible(s), vers une deuxième position, dans laquelle l'analyse dudit moyen de prélèvement (5, 50, 105, 205) et/ou la désolidarisation dudit moyen de prélèvement (5, 50, 105, 205) dudit contenant (2, 2', 102, 202) est/sont possible(s), et
dans lequel ladite partie dudit moyen de prélèvement (5, 50, 105, 205) et/ou ladite partie du moyen d'entrave d'analyse (140, 240) est/sont connectée(s) audit conteneur (6, 6', 106, 206) de sorte que l'ouverture dudit conteneur (6, 6', 106, 206) est mécaniquement inductible par le passage de ladite première position vers ladite deuxième position.

2. Dispositif (1, 100, 200) selon la revendication 1, dans lequel ledit moyen de prélèvement (5, 105, 205) est adapté pour être analysé dans ladite deuxième position, ledit moyen de prélèvement (5, 105, 205) comprenant au moins un moyen de capture d'information biologique et/ou physico-chimique adapté pour être mis en contact avec ledit échantillon biologique dans ladite première position et analysé dans ladite deuxième position.

3. Dispositif (1, 100, 200) selon la revendication 2, dans lequel ledit moyen de capture est adapté pour être analysé, dans ladite deuxième position, par lecture visuelle ou optique, de préférence par lecture visuelle, ledit moyen d'entrave d'analyse (21, 140, 240) étant un élément entravant au moins partiellement, de préférence totalement, la transmission de l'information lumineuse entre ledit moyen de prélèvement (5, 105, 205) et l'oeil humain ou ledit moyen de lecture optique dans ladite première position.

4. Dispositif (1, 100, 200) selon la revendication 3, dans lequel l'élément entravant au moins partiellement, de préférence totalement, la transmission de l'information lumineuse entre ledit moyen de prélèvement (5, 105, 205) et l'oeil humain ou ledit moyen de lecture optique dans ladite première position consiste en au moins une zone opaque au niveau d'au moins une paroi (21) dudit contenant (2, 102, 202) et/ou consiste en un cache mobile (140, 240), de préférence positionné à l'intérieur dudit contenant (2, 102, 202).

5. Dispositif (1, 100, 200) selon l'une des revendications 2 à 4, dans lequel ledit moyen de capture d'information biologique et/ou physico-chimique consiste en un moyen de capture de micro-organisme(s) tel qu'un support de capture apte à capturer le ou les micro-organisme(s) recherché(s), ledit moyen de capture de micro-organismes étant apte à être analysé, après capture du ou des micro-organisme(s) recherché(s), par au moins un moyen d'analyse.

6. Dispositif (1 , 1') selon la revendication 1, dans lequel ledit moyen de prélèvement est adapté pour être placé dans une position de désolidarisation, ledit moyen de prélèvement consistant en une membrane flexible (12, 12') connectée audit contenant (2, 2'), ladite membrane flexible (12, 12') étant adaptée pour être invaginée à l'intérieur du contenant (2, 2') dans ladite première position et au moins partiellement évaginée à l'extérieur du contenant (2, 2') dans ladite deuxième position.

7. Dispositif (1, 1') selon la revendication 6, dans lequel ladite membrane flexible (12, 12') comprend au moins un moyen de capture d'information biologique et/ou physico-chimique, ledit moyen de capture d'information biologique et/ou physico-chimique étant positionné sur la surface de ladite membrane flexible (12, 12') dans une position adaptée pour être en contact avec l'échantillon biologique dans ladite première position et n'être plus en contact avec ledit échantillon biologique dans ladite deuxième position.

8. Dispositif (1, 1', 100, 200) selon l'une des revendications 1 à 7, dans lequel ledit conteneur (6, 6', 106, 206) comprend également au moins un marqueur tel qu'un marqueur coloré permettant de vérifier le relargage dudit agent désinfectant à l'intérieur du contenant (2, 2', 102, 202) après ouverture dudit conteneur (6, 6', 106, 206).

9. Dispositif (1, 1', 100, 200) selon l'une des revendications 1 à 8, dans lequel ledit moyen de prélèvement (5, 50, 105, 205) est connecté audit contenant (2, 2', 102, 202) par un moyen d'inviolabilité, tel qu'une bague d'inviolabilité (30).

10. Dispositif (1, 1', 100, 200) selon l'une des revendications 1 à 9, comprenant une pluralité de moyens de prélèvement (5, 50, 105, 205) identiques ou différents positionnés dans l'enceinte dudit contenant (2, 2', 102, 202), les moyens de prélèvement (5, 50, 105, 205) étant connectés ensemble de telle manière que le passage de ladite première position vers ladite deuxième position d'au moins un des moyens de prélèvement (5, 50, 105, 205) provoque, dans le même temps, le passage de ladite première position vers ladite deuxième position des autres moyens de prélèvement (5, 50, 105, 205), induisant ainsi l'ouverture dudit conteneur (6, 6', 106, 206).

11. Procédé permettant d'assurer la désinfection de tout ou partie de l'intérieur d'un contenant (2, 2') adapté pour recevoir au moins un échantillon biologique, ledit procédé mettant en oeuvre le dispositif (1, 1') selon l'une des revendications 1 à 10, ledit procédé comprenant les étapes suivantes :
a) introduire, par ledit moyen de remplissage, au moins un échantillon biologique au sein dudit contenant (2, 2'),
b) fermer ledit moyen de remplissage, de préférence de manière hermétique, à l'aide dudit moyen de fermeture (4, 4'),
c) exercer une force, par exemple de type traction ou rotation, sur au moins une partie dudit moyen de prélèvement (5, 50) et/ou au moins une partie dudit moyen d'entrave d'analyse (140, 240) afin de passer de ladite première position vers ladite deuxième position, en vue d'analyser et/ou de désolidariser ledit moyen de prélèvement (5, 50),
d) effectuer, dans ladite deuxième position, l'analyse dudit moyen de prélèvement (5, 50) à l'aide d'au moins un moyen d'analyse positionné à l'extérieur dudit contenant (2, 2'), et/ou
d') désolidariser, dans ladite deuxième position, ledit moyen de prélèvement (5, 50), de préférence par séparation hermétique, par exemple par thermoscellage et coupure, en vue d'analyser tout ou partie dudit échantillon biologique présent au niveau dudit moyen de prélèvement (5, 50) lors d'une étape d'analyse e) réalisée dans un autre contenant,
le passage de ladite première position vers ladite deuxième position induisant mécaniquement l'ouverture dudit conteneur (6, 6'), permettant ainsi d'assurer la désinfection de tout ou partie dudit contenant (2, 2').

12. Procédé selon la revendication 11, dans lequel, à l'étape c), l'on exerce une force de traction sur au moins un moyen de préhension (8) connecté à ladite partie dudit moyen de prélèvement (5, 50) et/ou à ladite partie dudit moyen d'entrave d'analyse (140, 240).

13. Procédé selon la revendication 11 ou 12, dans lequel, à l'étape c), l'on exerce une force, par exemple de type traction ou rotation, sur au moins une partie dudit moyen de prélèvement (5, 50) et/ou au moins une partie dudit moyen d'entrave d'analyse (140, 240), afin de passer de ladite première position, dans laquelle l'analyse dudit moyen de prélèvement (5, 50) est malaisée ou impossible, de préférence impossible, vers ladite deuxième position dans laquelle l'analyse dudit moyen de prélèvement est possible, en vue d'effectuer, à l'étape d), l'analyse dudit moyen de prélèvement sans sortir ledit moyen de prélèvement dudit dispositif (1, 1').

14. Procédé selon l'une des revendications 11 à 13, dans lequel l'échantillon biologique est susceptible de contenir au moins un micro-organisme à analyser, ledit procédé comprenant, lors de l'étape a) ou postérieurement à cette dernière, l'étape suivante :
a') mettre en contact ledit échantillon biologique avec au moins un moyen de culture (3, 3') adapté pour permettre la croissance du ou des micro-organisme(s) à analyser, ledit procédé comprenant en outre, après l'étape b) et avant l'étape c), l'étape suivante :
b') éventuellement placer ledit dispositif (1, 1') dans des conditions aptes à permettre la croissance du ou des micro-organisme(s) à analyser.

15. Procédé selon l'une des revendications 11 à 14, dans lequel ledit moyen de prélèvement (5, 105, 205) comprend au moins un moyen de capture de micro-organisme(s), tel qu'un support de capture apte à capturer le ou les micro-organisme(s) recherché(s), adapté pour être analysé à l'étape d) et/ou à l'étape e).

16. Procédé selon l'une des revendications 11 à 15, dans lequel, à l'étape d), l'analyse dudit moyen de prélèvement (5, 105, 205) est réalisée par lecture visuelle ou optique.

17. Procédé selon la revendication 16, dans lequel ledit échantillon biologique est mis en contact avec un système de révélation apte à permettre ou à favoriser ladite lecture visuelle ou optique par exemple lors de l'étape a).

18. Procédé selon l'une des revendications 11 à 17, ledit procédé comprenant successivement les étapes d) et d'), dans lequel l'étape d'analyse e) est une étape de confirmation d'analyse visant à confirmer ou infirmer le résultat d'analyse obtenu à l'étape d).

## Patentansprüche

1. Eine Vorrichtung (1, 1', 100, 200), die zur Aufnahme mindestens einer biologischen Probe angepasst ist, wobei die Vorrichtung (1, 1', 100, 200) Folgendes beinhaltet:
- mindestens einen vorzugsweise dichten Behälter (2, 2', 102, 202), der angepasst ist zur Aufnahme der biologischen Probe, wobei der Behälter (2, 2', 102, 202) mindestens ein Füllmittel, wie etwa eine Öffnung, und mindestens ein Schließmittel (4, 4', 104), das ermöglicht, das Füllmittel vorzugsweise auf hermetische Weise zu schließen, beinhaltet,
- mindestens ein Probennahmemittel (5, 50, 105, 205), das in der Kammer des Behälters (2, 2', 102, 202) ortsfest oder beweglich positioniert ist, wobei das Probennahmemittel (5, 50, 105, 205) zum Entnehmen von biologischen und/oder physikalisch-chemischen Informationen aus der biologischen Probe angepasst ist, wobei das Probennahmemittel (5, 50, 105, 205) angepasst ist, um nach dem Entnehmen der biologischen und/oder physikalisch-chemischen Informationen analysiert und/oder in einer Freigabeposition angeordnet zu werden, und
- mindestens ein Gefäß (6, 6', 106, 206), wie etwa ein Beutel oder ein Kompartiment, der/das mindestens ein Desinfektionsmittel beinhaltet, wobei das Desinfektionsmittel nach dem Öffnen des Gefäßes (6, 6', 106, 206) in den Behälter (2, 2', 102, 202) freisetzbar ist
- wenn das Probennahmemittel (5, 50, 105, 205) angepasst ist, nach der Entnahme der biologischen und/oder physikalisch-chemischen Informationen analysiert zu werden, wobei mindestens ein Analyseeinschränkungsmittel (21, 140, 240), verschieden von dem Probennahmemittel (5, 50, 105, 205), ortsfest (21) oder beweglich (140, 240), die Analyse des Probennahmemittels (5, 50, 105, 205) teilweise oder vollständig, vorzugsweise vollständig, einschränkt,
wobei mindestens ein Teil des Probennahmemittels (5, 50, 105, 205) und/oder mindestens ein Teil des Analyseeinschränkungsmittels (140, 240) angepasst ist/sind, um von einer ersten Position, in welcher die Analyse von dem Probennahmemittel (5, 50, 105, 205) und/oder die Freigabe des Probennahmemittels (5, 50, 105, 205) des Behälters (2, 2', 102, 202) schwierig oder unmöglich ist/sind, vorzugsweise unmöglich, in Richtung einer zweiten Position, in welcher die Analyse des Probennahmemittels (5, 50, 105, 205) und/oder die Freigabe des Probennahmemittels (5, 50, 105, 205) des Behälters (2, 2', 102, 202) möglich ist/sind, überzugehen, und
wobei der Teil des Probennahmemittels (5, 50, 105, 205) und/oder der Teil des Analyseeinschränkungsmittels (140, 240) derart mit dem Gefäß (6, 6', 106, 206) verbunden ist/sind, dass die Öffnung des Gefäßes (6, 6', 106, 206) mechanisch durch den Übergang von der ersten Position in Richtung der zweiten Position induziert werden kann.

2. Vorrichtung (1, 100, 200) gemäß Anspruch 1, wobei das Probennahmemittel (5, 105, 205) zur Analyse in der zweiten Position angepasst ist, wobei das Probennahmemittel (5, 105, 205) mindestens ein Mittel zum Erfassen von biologischen und/oder physikalisch-chemischen Informationen beinhaltet, die angepasst sind, um mit der biologischen Probe in der ersten Position in Kontakt gebracht zu werden und in der zweiten Position analysiert zu werden.

3. Vorrichtung (1, 100, 200) gemäß Anspruch 2, wobei das Erfassungsmittel angepasst ist, um in der zweiten Position durch visuelles oder optisches Auslesen, vorzugsweise durch visuelles Auslesen analysiert zu werden, wobei das Analyseeinschränkungsmittel (21, 140, 240) ein Element ist, das die Übertragung von Lichtinformationen zwischen dem Probennahmemittel (5, 105, 205) und dem menschlichen Auge oder dem Mittel zum optischen Auslesen in der ersten Position mindestens teilweise, vorzugsweise vollständig einschränkt.

4. Vorrichtung (1, 100, 200) gemäß Anspruch 3, wobei das Element, das die Übertragung von Lichtinformationen zwischen dem Probennahmemittel (5, 105, 205) und dem menschlichen Auge oder dem Mittel zum optischen Auslesen in der ersten Position mindestens teilweise, vorzugsweise vollständig einschränkt, aus mindestens einem undurchsichtigen Bereich an mindestens einer Wand (21) des Behälters (2, 102, 202) besteht und/oder aus einem beweglichen Abdeckelement (140, 240), die vorzugsweise im Inneren des Behälters (2, 102, 202) positioniert ist, besteht.

5. Vorrichtung (1, 100, 200) gemäß einem der Ansprüche 2 bis 4, wobei das Mittel zum Erfassen biologischer und/oder physikalisch-chemischer Informationen aus einem Mittel zum Erfassen von einem Mikroorganismus/Mikroorganismen besteht, wie etwa einem Erfassungsträger, der zum Erfassen des gesuchten Mikroorganismus oder der gesuchten Mikroorganismengeeignet ist, wobei das Mittel zum Erfassen von Mikroorganismen geeignet ist, nach dem Erfassen des gesuchten Mikroorganismus oder der gesuchten Mikroorganismen durch mindestens ein Analysemittel analysiert zu werden.

6. Vorrichtung (1, 1') gemäß Anspruch 1, wobei das Probennahmemittel angepasst ist, um in einer Freigabeposition angeordnet zu werden, wobei das Probennahmemittel aus einer flexiblen Membran (12, 12') besteht, die mit dem Behälter (2, 2') verbunden ist, wobei die flexible Membran (12, 12') angepasst ist, um innerhalb des Behälters (2, 2') in der ersten Position invaginiert zu werden und außerhalb des Behälters (2, 2') in der zweiten Position mindestens teilweise evaginiert zu werden.

7. Vorrichtung (1, 1') gemäß Anspruch 6, wobei die flexible Membran (12, 12') mindestens ein Mittel zum Erfassen biologischer und/oder physikalisch-chemischer Informationen beinhaltet, wobei das Mittel zum Erfassen biologischer und/oder physikalisch-chemischer Informationen auf der Oberfläche der flexiblen Membran (12, 12') in einer Position positioniert ist, die angepasst ist, um mit der biologischen Probe in der ersten Position in Kontakt zu stehen und in der zweiten Position nicht mehr mit der biologischen Probe in Kontakt zu stehen.

8. Vorrichtung (1, 1', 100, 200) gemäß einem der Ansprüche 1 bis 7, wobei das Gefäß (6, 6', 106, 206) auch mindestens eine Markierung, wie etwa eine farbige Markierung, beinhaltet, die ermöglicht, die Freigabe des Desinfektionsmittels innerhalb des Behälters (2, 2', 102, 202), nachdem das Gefäß (6, 6', 106, 206) geöffnet wurde, zu überprüfen.

9. Vorrichtung (1, 1', 100, 200) gemäß einem der Ansprüche 1 bis 8, wobei das Probennahmemittel (5, 50, 105, 205) mit dem Behälter (2, 2', 102, 202) durch ein manipulationssicheres Mittel, wie etwa einen manipulationssicheren Ring (30), verbunden ist.

10. Vorrichtung (1, 1', 100, 200) gemäß einem der Ansprüche 1 bis 9, beinhaltend eine Vielzahl von identischen oder unterschiedlichen Probennahmemitteln (5, 50, 105, 205), die in der Kammer des Behälters (2, 2', 102, 202) positioniert sind, wobei die Probennahmemittel (5, 50, 105, 205) derart miteinander verbunden sind, dass der Übergang von mindestens einem der Probennahmemittel (5, 50, 105, 205) von der ersten Position in Richtung der zweiten Position gleichzeitig den Übergang von den anderen Probennahmemitteln (5, 50, 105, 205) von der ersten Position in Richtung der zweiten Position bewirkt, wodurch das Öffnen des Gefäßes (6, 6', 106, 206) induziert wird.

11. Verfahren, das ermöglicht, dass die Desinfektion des gesamten oder eines Teils des Inneren eines Behälters (2, 2'), der angepasst ist, um mindestens eine biologische Probe aufzunehmen, sichergestellt wird, wobei das Verfahren die Vorrichtung (1, 1') gemäß einem der Ansprüche 1 bis 10 einsetzt, wobei das Verfahren die folgenden Schritte beinhaltet:
a) Einführen mindestens einer biologischen Probe über das Füllmittel in den Behälter (2, 2'),
b) Schließen des Füllmittels, vorzugsweise auf hermetische Weise, mithilfe des Schließmittels (4, 4'),
c) Ausüben einer Kraft, zum Beispiel vom Typ Zug oder Drehung, auf mindestens einen Teil des Probennahmemittels (5, 50) und/oder mindestens einen Teil des Analyseeinschränkungsmittels (140, 240), um von der ersten Position in die zweite Position überzugehen, mit dem Ziel, das Probennahmemittel (5, 50) zu analysieren und/oder freizugeben,
d) Ausführen der Analyse des Probennahmemittels (5, 50) in der zweiten Position mithilfe mindestens eines außerhalb des Behälters (2, 2') positionierten Analysemittels und/oder
d') Freigeben des Probennahmemittels (5, 50) in der zweiten Position, vorzugsweise durch hermetische Trennung, zum Beispiel durch Heißsiegeln und Schneiden, mit dem Ziel, die gesamte oder einen Teil der auf dem Probennahmemittel (5, 50) vorhandenen biologischen Probe zu analysieren, während in einem anderen Behälter ein Analyseschritt e) durchgeführt wird,
wobei der Übergang von der ersten Position in Richtung der zweiten Position das mechanische Öffnen des Gefäßes (6, 6') induziert, wodurch ermöglicht wird, die Desinfektion des gesamten oder eines Teils des Behälters (2, 2') sicherzustellen.

12. Verfahren gemäß Anspruch 11, wobei in Schritt c) eine Zugkraft auf mindestens ein Greifmittel (8) ausgeübt wird, das mit dem Teil des Probennahmemittels (5, 50) und/oder mit dem Teil des Analyseeinschränkungsmittels (140, 240) verbunden ist.

13. Verfahren gemäß Anspruch 11 oder 12, wobei in Schritt c) auf mindestens einen Teil des Probennahmemittels (5, 50) und/oder mindestens einen Teil des Analyseeinschränkungsmittels (140, 240) eine Kraft, zum Beispiel vom Typ Zug oder Drehung, ausgeübt wird, um von der ersten Position, in der die Analyse des Probennahmemittels (5, 50) schwierig oder unmöglich ist, vorzugsweise unmöglich, in Richtung der zweiten Position, in der die Analyse des Probenahmemittels möglich ist, überzugehen, mit dem Ziel, in Schritt d) die Analyse des Probennahmemittels durchzuführen, ohne das Probennahmemittel aus der Vorrichtung (1, 1') zu entfernen.

14. Verfahren gemäß einem der Ansprüche 11 bis 13, wobei die biologische Probe vermutlich mindestens einen zu analysierenden Mikroorganismus enthält, wobei das Verfahren während Schritt a) oder im Anschluss an Letztgenannten, den folgenden Schritt beinhaltet:
a') In-Kontakt-Bringen der biologischen Probe mit mindestens einem Kulturmittel (3, 3'), das angepasst ist, um des Wachstums des zu analysierenden Mikroorganismus/der zu analysierenden Mikroorganismen zu ermöglichen, wobei das Verfahren ferner nach Schritt b) und vor Schritt c) den folgenden Schritt beinhaltet:
b') optionales Anordnen der Vorrichtung (1, 1') unter Bedingungen, die geeignet sind, das Wachstum des/der zu analysierenden Mikroorganismus/Mikroorganismen zu ermöglichen.

15. Verfahren gemäß einem der Ansprüche 11 bis 14, wobei das Probennahmemittel (5, 105, 205) mindestens ein Mittel zum Erfassen des Mikroorganismus/der Mikroorganismen beinhaltet, wie etwa einen Erfassungsträger, der zum Erfassen des gesuchten Mikroorganismus/der gesuchten Mikroorganismen geeignet ist, das angepasst ist, um in Schritt d) und/oder Schritt e) analysiert zu werden.

16. Verfahren gemäß einem der Ansprüche 11 bis 15, wobei in Schritt d) die Analyse des Probennahmemittels (5, 105, 205) durch visuelles oder optisches Auslesen durchgeführt wird.

17. Verfahren gemäß Anspruch 16, wobei die biologische Probe mit einem Nachweissystem in Kontakt gebracht wird, das geeignet ist, das visuelle oder optische Auslesen beispielsweise während Schritt a) zu ermöglichen oder zu begünstigen.

18. Verfahren gemäß einem der Ansprüche 11 bis 17, wobei das Verfahren nacheinander die Schritte d) und d') beinhaltet, wobei der Analyseschritt e) ein Analysebestätigungsschritt ist, der das Ergebnis der in Schritt d) erhaltenen Analyse bestätigen oder ungültig machen soll.

## Claims

1. Device (1, 1', 100, 200) suitable for receiving at least one biological sample, said device (1, 1', 100, 200) comprising:
- at least one container (2, 2', 102, 202), preferably leak-proof, suitable for receiving said biological sample, said container (2, 2', 102, 202) comprising at least one filling means, such as an opening, and at least one closing means (4, 4', 104) which makes it possible to close said filling means, preferably hermetically,
- at least one sampling means (5, 50, 105, 205) positioned inside the enclosure of said container (2, 2', 102, 202), stationary or movable, said sampling means (5, 50, 105, 205) being suitable for sampling biological and/or physicochemical information from said biological sample, said sampling means (5, 50, 105, 205) being suitable for being analysed after said biological and/or physicochemical information is sampled and/or for being placed in a release position, and
- at least one receptacle (6, 6', 106, 206), such as a pouch or a compartment, comprising at least one disinfecting agent, said disinfecting agent being able to be released inside the container (2, 2', 102, 202) after said receptacle (6, 6', 106, 206) is opened,
- when said sampling means (5, 50, 105, 205) is suitable for being analysed after said biological and/or physicochemical information is sampled, at least one analysis restricting means (21, 140, 240) separate from said sampling means (5, 50, 105, 205), stationary (21) or movable (140, 240), partially or completely restricting, preferably completely, the analysis of said sampling means (5, 50, 105, 205),
wherein at least a part of said sampling means (5, 50, 105, 205) and/or at least a part of the analysis restricting means (140, 240) are/is suitable for shifting from a first position, wherein the analysis of said sampling means (5, 50, 105, 205) and/or the release of said sampling means (5, 50, 105, 205) from said container (2, 2', 102, 202) are/is difficult or impossible, preferably impossible, to a second position, wherein the analysis of said sampling means (5, 50, 105, 205) and/or the release of said sampling means (5, 50, 105, 205) from said container (2, 2', 102, 202) are/is possible, and
wherein said part of said sampling means (5, 50, 105, 205) and/or said part of the analysis restricting means (140, 240) are/is connected to said receptacle (6, 6', 106, 206) such that the opening of said receptacle (6, 6', 106, 206) is mechanically inducible by shifting from said first position to said second position.

2. Device (1, 100, 200) according to Claim 1, wherein said sampling means (5, 105, 205) is suitable for being analysed in said second position, said sampling means (5, 105, 205) comprising at least one biological and/or physicochemical information capture means suitable for being contacted with said biological sample in said first position and analysed in said second position.

3. Device (1, 100, 200) according to Claim 2, wherein said capture means is suitable for being analysed, in said second position, by visual or optical reading, preferably by visual reading, said analysis restricting means (21, 140, 240) being a component restricting at least partially, preferably completely, the transmission of light information between said sampling means (5, 105, 205) and the human eye or said optical reading means in said first position.

4. Device (1, 100, 200) according to Claim 3, wherein the component restricting at least partially, preferably completely, the transmission of light information between said sampling means (5, 105, 205) and the human eye or said optical reading means in said first position consists of at least one opaque area on at least one wall (21) of said container (2, 102, 202) and/or consists of a movable cover (140, 240) preferably positioned inside said container (2, 102, 202).

5. Device (1, 100, 200) according to one of Claims 2 to 4, wherein said biological and/or physicochemical information capture means consists of a microorganism(s) capture means, such as a capture substrate capable of capturing the microorganism(s) sought, said microorganism(s) capture means being capable of being analysed, after capture of the microorganism(s) sought, by at least one analysis means.

6. Device (1, 1') according to Claim 1, wherein said sampling means is suitable for being placed in a release position, said sampling means consisting of a flexible membrane (12, 12') connected to said container (2, 2'), said flexible membrane (12, 12') being suitable for being invaginated inside the container (2, 2') in said first position and at least partially evaginated outside the container (2, 2') in said second position.

7. Device (1, 1') according to Claim 6, wherein said flexible membrane (12, 12') comprises at least one biological and/or physicochemical information capture means, said biological and/or physicochemical information capture means being positioned on the surface of said flexible membrane (12, 12') in a position suitable for being in contact with the biological sample in said first position and for no longer being in contact with said biological sample in said second position.

8. Device (1, 1', 100, 200) according to one of Claims 1 to 7, wherein said receptacle (6, 6', 106, 206) comprises also at least one marker, such as a coloured marker which makes it possible to verify the release of said disinfecting agent inside the container (2, 2', 102, 202) after said receptacle (6, 6', 106, 206) is opened.

9. Device (1, 1', 100, 200) according to one of Claims 1 to 8, wherein said sampling means (5, 50, 105, 205) is connected to said container (2, 2', 102, 202) by a tamper-evident means, such as a tamper-evident ring (30).

10. Device (1, 1', 100, 200) according to one of Claims 1 to 9, comprising a plurality of identical or different sampling means (5, 50, 105, 205) positioned inside the enclosure of said container (2, 2', 102, 202), the sampling means (5, 50, 105, 205) being joined together in such a way that the shifting from said first position to said second position of at least one of the sampling means (5, 50, 105, 205) causes, simultaneously, the shifting from said first position to said second position of the other sampling means (5, 50, 105, 205), thus inducing the opening of said receptacle (6, 6', 106, 206).

11. Process for ensuring the disinfection of all or part of the inside of a container (2, 2') suitable for receiving at least one biological sample, said process implementing the device (1, 1') according to one of Claims 1 to 10, said process comprising the following steps:
a) introducing, via said filling means, at least one biological sample into said container (2, 2'),
b) closing said filling means, preferably hermetically, by means of said closing means (4, 4'),
c) exerting force, for example of the pulling or rotational type, on at least a part of said sampling means (5, 50) and/or at least a part of said analysis restricting means (140, 240) so as to shift from said first position to said second position, in order to analyse and/or release said sampling means (5, 50),
d) carrying out, in said second position, the analysis of said sampling means (5, 50) by means of at least one analysis means positioned outside said container (2, 2'), and/or
d') releasing, in said second position, said sampling means (5, 50), preferably by hermetic separation, for example by heat sealing and cutting, in order to analyse all or part of said biological sample present on said sampling means (5, 50) during an analysis step e) performed in another container,
shifting from said first position to said second position, mechanically inducing the opening of said receptacle (6, 6'), thus ensuring the disinfection of all or part of said container (2, 2').

12. Process according to Claim 11, wherein, in step c), a pulling force is exerted on at least one gripping means (8) connected to said part of said sampling means (5, 50) and/or to said part of said analysis restricting means (140, 240).

13. Process according to Claim 11 or 12, wherein, in step c), a force, for example of the pulling or rotational type, is exerted on at least a part of said sampling means (5, 50) and/or at least a part of said analysis restricting means (140, 240) so as to shift from said first position, wherein the analysis of said sampling means (5, 50) is difficult or impossible, preferably impossible, to said second position, wherein the analysis of said sampling means is possible, in order to carry out, in step d), the analysis of said sampling means without removing said sampling means from said device (1, 1').

14. Process according to one of Claims 11 to 13, wherein the biological sample is likely to contain at least one microorganism to be analysed, said process comprising, during step a) or subsequent to this step, the following step:
a') contacting said biological sample with at least one culture means (3, 3') suitable for allowing growth of the microorganism(s) to be analysed, said process further comprising, after step b) and before step c), the following step:
b') optionally placing said device (1, 1') in conditions capable of allowing growth of the microorganism(s) to be analysed.

15. Process according to one of Claims 11 to 14, wherein said sampling means (5, 105, 205) comprises at least one microorganism(s) capture means, such as a capture substrate capable of capturing the microorganism(s) sought, suitable for being analysed in step d) and/or step e).

16. Process according to one of Claims 11 to 15, wherein, in step d), the analysis of said sampling means (5, 105, 205) is performed by visual or optical reading.

17. Process according to Claim 16, wherein said biological sample is contacted with a detection system capable of allowing or promoting said visual or optical reading, for example during step a).

18. Process according to one of Claims 11 to 17, said process successively comprising steps d) and d'), wherein analysis step e) is an analysis confirmation step aiming to confirm or invalidate the analysis result obtained in step d).
